# EUROPÄISCHE PATENTSCHRIFT

(11) **EP 2 696 783 B1**
(45) Veröffentlichungstag und Bekanntmachung des Hinweises auf die Patenterteilung: **30.03.2016**
(21) Anmeldenummer: 12714614.0
(22) Anmeldetag: 30.03.2012
(51) Int. Cl.: A61B 17/70

(54) **CHIRURGISCHES IMPLANTAT ZUM ERWEITERN EINES WIRBELKANALS**
SURGICAL IMPLANT FOR WIDENING A VERTEBRAL CANAL
IMPLANT CHIRURGICAL DESTINÉ À ÉLARGIR LE CANAL VERTÉBRAL

(30) Priorität: 12.04.2011 DE 102011001996
(43) Veröffentlichungstag der Anmeldung: 19.02.2014
(73) Patentinhaber: Aesculap AG, 78532 Tuttlingen (DE)
(72) Erfinder: BEGER, Jens, 78532 Tuttlingen (DE); LINKE, Ralph, 78256 Steisslingen (DE); SUCHOMEL, Petr, 460063 Liberec (CZ); KLINGSEIS, Susanne, 88400 Biberach (DE)
(74) Vertreter: Hoeger, Stellrecht & Partner Patentanwälte mbB
(86) Internationale Anmeldenummer: PCT/EP2012/055843
(87) Internationale Veröffentlichungsnummer: WO 2012/139905

(56) Entgegenhaltungen:
- WO-A1-03/020143
- WO-A2-2009/025884
- JP-A- 2000 139 970
- JP-A- 2001 149 392
- US-A- 5 980 572
- US-A1- 2009 198 278
- US-A1- 2010 161 056

## Beschreibung

Die Erfindung betrifft ein chirurgisches Implantat zur Verwendung in einem Verfahren zum Erweitern eines Wirbelkanals eines Wirbels der Wirbelsäule, insbesondere der Laminoplastie und der Laminektomie.

Die Wirbelkanäle der Wirbel der Wirbelsäule bilden den so genannten Rückenmarkskanal oder Spinalkanal, in dem das Rückenmark, umhüllt von der Rückenmarkshaut, aufgenommen ist.

Das Rückenmark als Teil des zentralen Nervensystems kann in seiner Funktion beeinträchtigt werden, wenn z.B. beim Vorliegen einer Spinalkanalstenose Druck auf das Rückenmark ausgeübt wird, was mehrere Ursachen haben kann, beispielsweise das Vorliegen einer Spondylose oder einer Verknöcherung des hinteren Längsbandes.

Abhilfe geschaffen werden kann hier, indem der Wirbelkanal des oder der betroffenen Wirbel der Wirbelsäule vergrößert wird, so dass das Rückenmark mehr Raum zur Verfügung hat und somit dem Druck ausweichen kann.

Eine Übersicht über die bislang gängigen Therapiemöglichkeiten findet sich z.B. bei F. Meyer et al., Deutsches Ärzteblatt, Jg. 105, Heft 20, Seiten 366 bis 372. Neben den ventralen Verfahren kommen verschiedene dorsale Verfahren, nämlich die Laminektomie mit und ohne Fusion sowie die Laminoplastie, zum Einsatz. Gegebenenfalls können ventrale auch mit dorsalen Verfahren kombiniert zum Einsatz kommen.

Von den verschiedenen dorsalen Verfahren kommt die Laminoplastie mit den geringfügigsten Eingriffen in die Knochensubstanz aus.

Vorgeschlagen wurden bisher verschiedene Laminoplastie-Operationstechniken, von denen die beiden wichtigsten in der Literatur als so genannte Single-Door- oder Double-Door-Techniken beschrieben werden. Eine Übersicht hierüber und eine Bewertung der zu erwartenden Effekte bezüglich der Druckentlastung bzw. der Expansion des Spinalkanals ist beispielsweise in der Veröffentlichung von Wang, Xiang-Yang et al. in SPINE, Vol. 31, Nr. 24, 2006, Seiten 2863 bis 2870, enthalten.

Bei der sogenannten Single-Door-Technik, auch Open-Door-Technik genannt, wird die Lamina auf einer Seite des Wirbels mit einem Schnittspalt durchtrennt, während auf der anderen Seite der Lamina eine Kerbe ohne Durchtrennung des Wirbelbogens geschaffen wird.

Der Bereich des Wirbelbogens mit der Einkerbung fungiert bei der nachfolgenden Eröffnung des Wirbelkanals als eine Art Scharnier und erlaubt ein Öffnen des Wirbelbogens, das mit einem Bruch der Knochensubstanz verbunden ist. Der Wirbelbogen bleibt über die Knochenhaut und die Kollagenfasern der Knochensubstanz mit dem Wirbelkörper verbunden.

Bei der sogenannten Double-Door-Technik wird der Dornfortsatz eines Wirbels durchtrennt oder ganz entfernt und beidseits des Dornfortsatzes in der Lamina jeweils eine Kerbe gesetzt, wobei die die Kerben enthaltenden Bereiche des Wirbelbogens wieder als Scharniere fungieren. Die Eröffnung des Wirbelkanals erfolgt nun durch das Auseinanderschwenken der beiden Wirbelbogenabschnitte mit den gegebenenfalls noch vorhandenen zugehörigen Dornfortsatzteilen, wobei ebenfalls die Knochensubstanz im Bereich der Scharniere bricht. Die Wirbelbogenabschnitte bleiben auch hier über die Knochenhaut und die Kollagenfasern der Knochensubstanz mit dem Wirbelkörper verbunden.

Der Wirbelkanal der Wirbel wird bei beiden Techniken im eröffneten Zustand durch Implantate fixiert. Als Implantatmaterial kommt neben körpereigenem Knochenspan ein Hydroxyapatitspacer oder dgl. zum Einsatz.

Trotz des gegenüber anderen dorsalen Verfahren verminderten Eingriffs in die Knochensubstanz wird bei der Laminoplastie eine deutlich erhöhte Rate von anschließenden Nackenschmerzen noch als nachteilig empfunden, ebenso wie eine oft beobachtete Einschränkung der Beweglichkeit der Halswirbelsäule.

Aufgabe der Erfindung ist es, ein Implantat vorzuschlagen, mit dem eine Erweiterung des Wirbelkanals von Wirbeln mit geringeren Belastungen für den Patienten möglich ist.

Diese Aufgabe wird von einem chirurgischen Implantat mit den Merkmalen des Anspruchs 1 gelöst.

Vorteilhafte Ausgestaltungen des Implantats gehen aus dem Unetransprüchen hervor.

In vielen Fällen, insbesondere bei der Verwendung des erfindungsgemäßen Implantats im Rahmen der Laminoplastie, weist das erfindungsgemäße Implantat keilförmig gegeneinander geneigt angeordnete Kontaktflächen auf, wobei der Abstand der Kontaktflächen an einem dorsalen Endbereich des Implantatkörpers größer ist als am ventralen Endbereich, der im implantierten Zustand des Implantats benachbart zu Spinalkanal positioniert ist.

Die Konzeption des erfindungsgemäßen Implantats mit keilförmig gegeneinander geneigten Kontaktflächen ermöglicht eine großflächige Anlage des Implantats an den Schnittflächen der Knochensubstanz des Wirbelbogens. Dies begünstigt das präzise Einführen, einen sicheren Halt und dauerhafte Positionierung des Implantats im Schnittspalt.

Das erfindungsgemäße Implantat eignet sich insbesondere für ein Laminoplastie-Verfahren bei dem der Wirbelbogen eines Wirbels unter Bildung eines Schnittspalts, vorzugsweise im Bereich des Dornforsatzes, durchtrennt und der Schnittspalt unter elastisch/plastischer Verformung der Knochensubstanz der resultierenden Wirbelbogenabschnitts auf eine vorgegebene Spaltbreite aufgeweitet wird.

Ein wesentlicher Unterschied dieses Verfahrens zu den zuvor diskutierten Single- und Double-Door-Techniken der Laminoplastie liegt darin begründet, dass zum Einen nur noch ein einziger Trennschnitt am Wirbelbogen vorgenommen wird und eine Ablösung von Muskulatur von der Wirbelsäule in großem Umfang oder sogar im Wesentlichen ganz vermieden wird. Darüber hinaus unterbleibt ein Brechen der Knochensubstanz der Lamina, da die Aufweitung des Wirbelbogens mittels elastisch/plastischer Verformung desselben erzielt wird.

Aufgrund der inhärenten viskoelastischen Eigenschaften der Knochensubstanz kommt es bei diesem Verfahren zusätzlich zu der elastisch/plastischen Verformung, ohne dass dies jedoch zu einem Bruch der Knochensubstanz führt. Die Aufweitung wird bevorzugt nicht abrupt erzwungen, sondern allmählich ausgerührt, so dass die viskoelastischen Eigenschaften der Knochensubstanz zum Tragen kommen können. Dies kann kontinuierlich oder in kleinen Schritten von z.B. jeweils ca. 0,5 mm bis ca. 3 mm geschehen. Typischerweise lässt sich auf diese Weise beim C6-Wirbel eine Spaltaufweitung von ca. 15 mm innerhalb von ca. 10 sec bis ca. 5 min, insbesondere innerhalb ca. 30 sec bis ca. 3 min, weiter bevorzugt innerhalb ca. 1 min bis ca. 2 min erzielen.

Somit wird dem Problem der bisherigen Laminoplastietechniken entgegengewirkt, die zunächst zwingend weitreichende Eingriffe an der parallel zur Wirbelsäule verlaufenden Muskulatur notwendig machen, die auch in der nachoperativen Phase eine erhebliche Belastung für den Patienten bedeuten.

Dieses Verfahren kommt insbesondere mit einem erheblich geringeren chirurgischen Eingriff als der Stand der Technik und ohne eine seitliche Ablösung der Muskulatur von der Wirbelsäule aus.

Insbesondere wenn der Trennschnitt im Bereich des Dornfortsatzes vorgenommen wird, ist die Stelle am Wirbel sehr einfach zugänglich und die Muskulatur parallel zur Wirbelsäule kann im Wesentlichen unangetastet bleiben.

Der Schnittspalt kann mit sehr unterschiedlichen Werkzeugen erzeugt werden. Beispielsweise kann der Schnittspalt mit einem Ultraschall-Osteotom erzeugt werden, wobei Schnittspalte mit ca. 1 mm oder weniger resultieren.

Zur elastischen/plastischen Aufweitung des Schnittspalts werden bevorzugt spezielle Distraktionsinstrumente, insbesondere Distraktionszangen, verwendet, welche vorzugsweise mit Führungselementen für die sichere Platzierung eines Implantats ausgerüstet sind und weiter bevorzugt eine Messeinheit aufweisen, um die erzielte Schnittspaltaufweitung anzuzeigen.

Im Hinblick auf die weiteren Schritte wie z.B. den Einsatz des erfindungsgemäßen Implantats ist es bevorzugt, ein Distraktionsinstrument zu verwenden, dessen Spitzen oder Endbereiche abgewinkelt sind oder nach außen abstehende Vorsprünge tragen. Die abgewinkelten Spitzen oder Vorsprünge können unter die Lamina geschoben werden, d.h. sie liegen an der Spinalkanalseite der Lamina an, und sorgen so für einen sicheren Sitz während der Aufweitung des Schnittspalts.

Die Kräfte, die notwendig sind, um den Schnittspalt aufzuweiten, betragen typischerweise ca. 70 N bis ca. 200 N, insbesondere ca. 80 N bis ca. 150 N, für eine Aufweitung des Spalts um ca. 5 mm bis ca. 12 mm, jeweils bestimmt am Spinalkanal-seitigen Ende des Schnittspalts.

Die Schnittflächen des Schnittspalts werden in der aufgeweiteten Position über erfindungsgemäße Implantate fixiert.

Das Material, aus dem das erfindungsgemäße Implantat gefertigt wird, ist bevorzugt ein körperverträgliches Kunststoffmaterial, insbesondere PEEK, oder Titan oder eine Titanlegierung. Daneben eignen sich auch körpereigene Knochenspäne.

Implantate aus Kunststoff, insbesondere PEEK, werden bevorzugt an den mit dem Knochenmaterial in Berührung stehenden Oberflächen mit einer osteointegrativen Beschichtung versehen. Vorzugsweise wird diese Beschichtung als mikroporöse Reintitanschicht nach dem VPS-Verfahren (Plasmapore-Technik) oder als Hydroxyapatit-Schicht ausgeführt.

Die Kunststoff-Implantate sind gegenüber Titan-Implantaten bevorzugt, da sie zum MRT-Verfahren kompatibel sind. Dies ist insbesondere für die postoperative Phase von Bedeutung. Die MRT-Kompatibilität ist auch bei den oben beschriebenen osteointegrativ-beschichteten Kunststoffimplantaten gegeben.

Implantate aus Titan weisen bevorzugt eine poröse Struktur oder eine Gitternetzstruktur auf.

Das erfindungsgemäße Implantat weist eine Keilform auf, so dass eine möglichst vollflächige Anlage der Oberflächen des keilförmigen Körpers an den Schnittflächen des aufgeweiteten Schnittspalts erzielt wird, die nach der Bildung des Schnittspalts zunächst parallel, in Folge der elastischen Aufweitung des Schnittspalts aber keilförmig gegeneinander geneigt angeordnet sind.

Vorzugsweise definieren die keilförmig angeordneten Kontaktflächen des erfindungsgemäßen Implantats zwischen sich einen Winkel von ca. 5° bis ca. 45°, insbesondere 7° bis 30°.

Die erfindungsgemäßen Implantate lassen sich aber auch bei herkömmlichen Laminoplastie-Verfahren und auch der Laminektomie einsetzen.

Vorteilhafterweise werden an den Kontaktflächen der erfindungsgemäßen Implantate Führungselemente angeordnet. Die Führungselemente werden bevorzugt als Vor- oder Rücksprünge ausgebiidet.

Die Vor- oder Rücksprünge der bevorzugten erfindungsgemäßen Implantate können, insbesondere wenn an den Schnittflächen des Schnittspalts korrespondierende Rücksprünge vorgesehen werden, der Führung des Implantats beim Einsetzen in den Schnittspalt dienen und zum anderen die implantierten Implantate in ihrer Position stabilisieren.

Die Rücksprünge sind sowohl Implantat- als auch Schnittspalt-seitig bevorzugt als Nuten mit vorzugsweise in etwa halbzylindrischer Form ausgebildet, welche vorzugsweise parallel zur dorsal/ventral verlaufenden Längsachse des Implantatkörpers bzw. des Schnittspalts ausgerichtet sind.

Ein weiterer Aspekt besteht in der Führung der Implantatkörper beim Einsetzen in den Schnittspalt, wobei die Vorsprünge der Kontaktflächen der Implantatkörper gleitend in die Nuten der Schnittflächen eingreifen.

Die Nuten in den Schnittflächen können ferner als Positionierungshilfe für die Distraktionsinstrumente dienen. Sollen die Implantatkörper unmittelbar nach einer ausreichenden Distraktion der Wirbelbogenabschnitte in den Schnittspalt eingesetzt werden, so kann das Distraktionsinstrument partiell von den Nuten aufgenommen im Schnittspalt verbleiben und diesen in seiner aufgeweiteten Stellung halten, während Implantate, die bevorzugt an ihren Kontaktflächen korrespondierende Nuten aufweisen, durch das Distraktionsinstrument geführt in den Schnittspalt eingesetzt werden.

Die an den Kontaktflächen der Implantatkörper vorzugsweise vorhandenen Führungselemente in Form von Vorsprüngen können außerdem verwendet werden, um einen Anschlag an der Knochensubstanz im eingesetzten Zustand zu bilden, so dass das erfindungsgemäße Implantat nur in eine vorgegebene Tiefe in den Schnittspalt eingesetzt werden kann. Damit wird insbesondere sichergestellt, dass das Implantat mit seinem ventralen Endbereich nicht den Spinalkanal einengen kann. Diese Maßnahme sichert auch das Implantat in der Folgezeit in seiner korrekten Position.

Die Vorsprünge brauchen hierfür nicht unbedingt eine größere Ausdehnung in Längsrichtung des Implantatkörpers aufweisen. Eine längere Ausdehnung ist insbesondere dann allerdings wünschenswert, wenn die Führungsfunktion der Vorsprünge zusammen mit den Rücksprüngen auf Seiten der Schnittflächen der Schnittspalte zum Tragen kommen soll.

Bei einer weiter bevorzugten Ausführungsform der erfindungsgemäßen Implantate sind die Vor- oder Rücksprünge im dorsalen Endbereich in Sagittalrichtung außermittig angeordnet. Damit ist es möglich, die Vor- oder Rücksprünge an einer sagitalen Position des Schnittspalts anzuordnen, der mindestens partiell außerhalb des Bereichs der Dornfortsätze liegt. Ein einfacherer Zugang zu dem oft an der Stelle von Vorsprüngen auf Seiten des Implantatkörpers angeordneten weiteren Funktionsteilen, die im Weiteren noch besprochen werden sollen ist in einem solchen Fall gegeben.

Der Implantatkörper kann mit einem alternativen oder weiteren Führungselement an seinem ventralen Endbereich versehen sein, welches vorzugsweise so ausgebildet ist, dass es zwischen die Branchen eines Distraktionsinstruments eingeführt und entlang dieser Branchen in Richtung zum Schnittspalt des Wirbels geführt und dabei in den Schnittspalt eingesetzt werden kann.

Vorzugsweise wird die Höhe des Implantats an die Tiefe des Schnittspalts angepasst und ergibt so eine maximale Fläche für die Anlage des Implantats an den Schnittflächen und damit eine geringe Flächenpressung seitens der Knochensubstanz. Dies ist von Bedeutung auch im Hinblick darauf, dass die Implantate dauerhaft im Körper der Patienten verbleiben.

Erfindungsgemäß lassen sich die Implantate mit ihrem im Schnittspalt eingesetzten Implantatkörper an dem Knochenmaterial in verschiedener Weise festlegen.

Die zuvor angesprochene Ausbildung von Rücksprüngen in den Schnittflächen und von Vorsprüngen in den Kontaktflächen der erfindungsgemäßen Implantatkörper können verwendet werden, um einen Formschluss zwischen dem Schnittspalt bzw. der Knochensubstanz des Wirbels und dem Implantat zu schaffen.

Die aufgrund der elastisch/plastischen Aufweitung des Wirbelbogens auf den Implantatkörper wirkenden Kräfte führen darüber hinaus zu einem gewissen anfänglichen Kraftschluss. Dieser nimmt im Laufe der Zeit aufgrund der viskoelastischen Eigenschaften der Knochensubstanz jedoch ab.

Bevorzugt kann deshalb vorgesehen sein, den Implantatkörper mit zusätzlichen Befestigungsmitteln an der Knochensubstanz zu verankern.

Beispielsweise kann der erfindungsgemäße Implantatkörper eine oder mehrere Bohrungen aufweisen, durch die hindurch der Implantatkörper mittels Stiften oder Schrauben, die in die Knochensubstanz eingreifen, fixiert werden.

Eine weitere Alternative besteht darin, die Implantatkörper mit Nahtmaterial oder Drähten am Wirbelbogen zu fixieren. Ebenso lassen sich Metall- oder Kunststoffstege, die den Implantatkörper übergreifen, beidseits des Schnittspalts an der Knochensubstanz festlegen, um den Implantatkörper im Schnittspalt zu halten und zu sichern.

So weisen bevorzugte Implantate einen Implantatkörper mit einer oder mehreren Querbohrungen auf, die quer zur Längsrichtung des keilförmigen Implantatkörpers ausgerichtet sind. Diese Querbohrungen bilden Kanäle, durch die Nahtmaterial hindurchgeführt werden kann, das wirbelseitig beispielsweise mit den Dornfortsätzen verbunden werden kann. Solche Querbohrungen sind vorzugsweise parallel zu den Kontaktflächen oder auch senkrecht hierzu angeordnet.

Werden Implantate mit größerer Tiefe verwendet, so sind mehrere Querbohrungen über die Längsrichtung des Implantatkörpers verteilt von Vorteil, weil dann der ventralseitige Endbereich des Implantatkörpers mit dem Wirbelbogen verbunden werden kann, während der dorsale Endbereich beispielsweise abgebrochene Abschnitte des Dornfortsatzes separat fixiert halten kann.

Alternativ können die Querbohrungen auch dazu verwendet werden, um von außen, d.h. durch die Knochensubstanz hindurch tretende Fixierelemente, beispielsweise Schrauben, Dübel, etc. Implantat-seitig aufzunehmen. Hierfür braucht es dann keine Durchgangsbohrungen, sondern Sacklochbohrungen sind hierfür ausreichend. Bei der Herstellung der Implantatkörper aus einem PEEK-Material werden bevorzugt auch die Fixierelemente, insbesondere die Schrauben oder Dübel, ebenfalls aus PEEK-Material hergestellt.

Bei einer bevorzugten Ausführungsform der erfindungsgemäßen Implantate weist der Implantatkörper Bohrungen auf, die als Durchgangsbohrungen ausgebildet sind und welche vom dorsalen Endbereich ausgehend im spitzen Winkel zu den Kontaktflächen des keilförmigen Implantatkörpers ausgerichtet sind und vorzugsweise die Kontaktflächen durchsetzen. Diese Bohrungen bilden Führungen für Befestigungselemente, wie Dorne, Dübel und Schrauben oder Splinte, die Implantat-seitig in den Bohrungen geführt in das im implantierten Zustand angrenzende Knochenmaterial eingesetzt, eingeschraubt oder eingetrieben werden können.

Der Winkel zur sagittalen Symmetrieebene, in dem die Achsen der Durchgangsbohrungen ausgerichtet sind, beträgt vorzugsweise ca. 10° bis 60°, insbesondere ca. 15° bis ca. 45°.

Die Winkel werden unter dem Gesichtspunkt gewählt, um einen möglichst langen Bohrungs- oder Gewindeabschnitt in der Knochensubstanz des Wirbelbogens zu erzielen und gleichzeitig zu vermeiden, dass die in die Bohrung bzw. das Gewinde eingesetzten Befestigungselemente in den Spinalkanal eindringen. Je länger der Bohrungs- oder Gewindeabschnitt in der Knochensubstanz ausgebildet werden kann, desto sicherer lässt sich Implantat verankern.

Bei einer Keilform des Implantats mit einem Winkel der Kontaktflächen zueinander von beispielsweise 10° haben sich in vielen Fällen Winkel von ca. 20° bis ca. 30° für die Achsen der Durchgangsbohrungen als vorteilhaft erwiesen.

Andere bevorzugte erfindungsgemäße Implantate umfassen ein vorzugsweise in den Körper integriertes Verriegelungselement, welches nach dem Positionieren des Implantatkörpers im Schnittspalt aktiviert werden kann. Das Verriegelungselement wird bevorzugt aus demselben Typ Material hergestellt wie der damit bestückte Implantatkörper.

Als Verriegelungselemente können beispielsweise Knochenschrauben dienen, deren Flanken in einer Drehposition innerhalb des Implantatkörpers angeordnet sind und bei einer Drehung um ca. 90° über die Kontaktflächen des Implantatkörpers hinausragen und sich in die umgebende Knochensubstanz einschneiden.

Eine weitere Alternative zum Festlegen des Implantatkörpers im Schnittspalt besteht darin, eine form- oder stoffschlüssige Verbindung zwischen dem Implantatkörper und der umgebenden Knochensubstanz herzustellen. Für die form- oder stoffschlüssige Verbindung eignen sich plastifizierbare oder aushärtbare Materialien oder aber sogenannte Heißkleber, die sich mittels Ultraschall, Wärme, HF oder auch UV-Licht aktivieren lassen.

Die Vorsprünge auf Seiten des Implantats sind bevorzugt dann passend zu den Formen der Nuten halbzylindrisch ausgebildet, so dass eine möglichst großflächige Anlage auch im Bereich der Vor- und Rücksprünge von Knochenmaterial und Implantat gewährleistet ist.

Bevorzugt weisen die erfindungsgemäßen Implantate in ihrem Implantatkörper ein Drehlager für das Verriegelungselement auf.

Alternativ kann vorgesehen sein, dass eine sich dorsal öffnende Sacklochbohrung, die im Wesentlichen parallel zur Längsachse des Implantatkörpers verläuft vorgesehen ist, die ein Verriegelungselement aufnehmen kann, welches mit einem Außengewinde versehen ist und einen Außenumfang aufweist, der größer ist als der Abstand der Kontaktflächen am dorsalen Endbereich des Implantatkörpers. Wird das Verriegelungselement in die Sacklochbohrung eingeschraubt, welches dann vorzugsweise ein Innengewinde ausgebildet enthält, schneidet sich das Außengewinde des Verriegelungselements in die umgebende Knochensubstanz des Wirbelbogens ein und sichert so das erfindungsgemäße Implantat in einem korrekten Sitz im Schnittspalt des Wirbelbogens.

Bei einer weiteren alternativen Ausführungsform der vorliegenden Erfindung kann vorgesehen sein, dass im Implantatkörper des erfindungsgemäßen Implantats eine Fixiervorrichtung vorgesehen ist, die ein oder mehrere dornartige Arretierelemente umfasst. Für diese Arretierelemente, die im nicht implantierten Zustand in einer Ruhelage innerhalb des Implantatkörpers angeordnet sind, ist eine Betätigungsvorrichtung vorgesehen, die diese in eine Arbeitslage überführen kann, wobei dann die Spitzen der Arretierelemente aus der Ruhelage innerhalb der Kontur des Implantatkörpers in eine Arbeitslage aus Öffnungen, die vorzugsweise in den Kontaktflächen des Implantatkörpers vorgesehen sind, heraustreten und in die umgebende Knochensubstanz eindringen.

Alternativ kann vorgesehen sein, dass am Implantatkörper gelenkig angeordnete Rastelemente vorgesehen sind, die über eine Art Kulissenführung nach Einsetzen des Implantats in den Schnittspalt im Wesentlichen quer zu den Kontaktflächen nach außen verlagert und so in die umgebende Knochensubstanz eingepresst werden können. Zu diesem Zweck weisen die Arretierelemente vorzugsweise hinterschnittene Flächenbereiche auf, so dass sich ein sicherer Sitz des Implantats im Schnittspalt durch einen Formschluss ergibt. Bei dieser Ausführungsform wird eine Art Dübelprinzip realisiert.

Bei einer weiteren Ausfühungsform des erfindungsgemäßen Implantats kann vorgesehen sein, dass der Implantatkörper eine im Wesentlichen zentral angeordnete Durchgangsöffnung aufweist, welche vom dorsalen Endbereich zum ventralen Endbereich des Implantatkörpers reicht. Aus der Sicht des Chirurgen sind die Begriffe dorsal mit proximal und ventral mit distal gleichzusetzen.

Diese Durchgangsöffnung kann beispielsweise ein Betätigungselement einer Verankerungsvorrichtung aufnehmen, welche auf Seiten des ventralen Endbereichs des Implantatkörpers ein Flächenelement umfasst, welches in mindestens einer Richtung seiner Flächenausdehnung eine größere Breite aufweist als der Abstand der Kontaktflächen des Implantatkörpers am ventralen Endbereich. Die Haltevorrichtung reicht bevorzugt durch die zentrale Durchgangsöffnung zum dorsalen Endbereich, wo sie in ihrer Einsatzposition festlegbar ist.

Das Flächenelement ist bevorzugt elastisch verformbar und wird in einer ersten Stellung elastisch verformt am oder im Implantatkörper gehalten, so dass die Flächenausdehnung des verformten Flächenelements in Richtung des Abstandes der Kontaktflächen geringer ist als der Abstand der Kontaktflächen am ventralen Endbereich des Implantatkörper.

Im eingesetzten Zustand des erfindungsgemäßen Implantats wird dann das Flächenelement aus dieser ersten Stellung heraus bewegt und mittels einer Haltevorrichtung, vorzugsweise der in der Durchgangsbohrung verschieblich gehaltenen Haltevorrichtung gegen den ventralen Endbereich des Implantatkörpers gezogen und in dieser Stellung fixiert. Das elastisch verformte Flächenelement wird dabei eine größere Ausdehnung annehmen als es dem Abstand der Kontaktflächen des Implantatkörpers am ventralen Endbereich entspricht, so dass das Flächenelement nicht nur am ventralen Endbereich des Implantatkörpers, sondern auch an der umgebenden Knochensubstanz des Wirbelbogens anliegt. Auf diese Weise kann das keilförmige Implantat der vorliegenden Erfindung sicher in dem Schnittspalt des Wirbelbogens gehalten werden.

Bevorzugt werden die bei den zuvor beschriebenen erfindungsgemäßen Implantaten verwendeten Rastelemente, Verriegelungselemente, die Fixiervorrichtungen und deren Arretierelemente sowie die Haltevorrichtungen aus einem Material desselben Typs hergestellt wie der damit bestückte Implantatkörper, insbesondere aus PEEK-Material.

Bei einer weiteren bevorzugten Ausführungsform der Erfindung ist der Implantatkörper konisch oder Kegelstumpf-förmig ausgebildet. Der Schnittspalt des Wirbelbogens wird für dieses erfindungsgemäße Implantat vorzugsweise mit einer in die Schnittflächen eingreifenden Bohrung vorbereitet, so dass sich eine Führung für die Spitze des konischen Implantats beim Einsetzen desselben ergibt.

Weiter bevorzugt weist der konische Implantatkörper an seiner Oberfläche ein umlaufendes Außengewinde auf, das sich beim Einsetzen bzw. Einschrauben des Implantats in den Wirbelbogen in die anliegende Knochensubstanz einschneidet.

Bei diesem Typ des erfindungsgemäßen Implantats kann der Schnittspalt durch das Einschrauben des Implantats aufgeweitet werden.

Die Konizität des Implantatkörpers entspricht bevorzugt einem Kegelwinkel von ca. 5° bis 45°, insbesondere von ca. 7° bis ca. 30°.

Bei bevorzugten Implantaten der vorliegenden Erfindung wird der ventrale Endbereich des Implantatkörpers konvex ausgebildet, so dass Spinalkanal-seitig zusätzlicher Raum geschaffen wird, auch wenn die Kontaktflächen des Implantatkörpers sich im Wesentlichen an der gesamten Schnittfläche des Schnittspaltes abstützen.

Am dorsalen Endbereich des erfindungsgemäßen Implantats wird vorzugsweise ein Griffelement vorgesehen mit denen das Implantat einfach mit einem Werkzeug gegriffen und in den Schnittspalt eingesetzt werden kann.

Anstelle eines angeformten Griffelementes kann das Implantat auch Bohrungen aufweisen, die dorsalseitig offen sind und die im Wesentlichen parallel zur Längsrichtung des Implantatkörpers verlaufen. In diese Bohrungen können Halteelemente eingesetzt bzw. eingeschraubt werden und so das Implantat über Instrumente sicher und gegebenenfalls geführt in den Schnittspalt eingesetzt werden.

Bevorzugte Implantatkörper weisen an ihrem ventralen Endbereich einen Abstand der keilförmig angeordneten Kontaktflächen auf, der ca. 5 bis ca. 15 mm beträgt. Damit wird ein entsprechender Abstand der Schnittflächen des Schnittspalts über das erfindungsgemäße Implantat fixiert, der im Falle, dass der ventrale Endbereich des Implantats nicht bis ganz zum Spinalkanal reicht, auch etwas geringer sein kann, als der vorstehend genannte Abstand.

Diese und weitere Vorteile der Erfindung werden im Folgenden anhand der Zeichnung noch näher erläutert. Es zeigen im Einzelnen:
- Figuren 1A und 1B: einen Teil der Halswirbelsäule mit einem mit einem Schnittspalt versehenen Halswirbel in perspektivischer bzw. Draufsicht;
- Figur 2: den Halswirbel der Figur 1B beim Aufweiten des Schnittspalts;
- Figur 3: eine schematische Schnittansicht durch einen elastisch/plastisch aufgeweiteten Wirbelkanal;
- Figuren 4A und 4B: zwei Varianten einer ersten Ausführungsform einer ersten Grundform eines Implantats;
- Figur 5: den Halswirbel der Figur 1B mit aufgeweitetem Schnittspalt und einem eingesetzten Implantat der Figur 4B in Seitenansicht;
- Figuren 6A bis 6D: eine zweite Ausführungsform der ersten Grundform des Implantats in perspektivischer Darstellung, in Seitenansicht und Draufsicht sowie in einem Schnittspalt eingesetzt;
- Figuren 7A bis 7F: eine dritte Ausführungsform der ersten Grundform des Implantats in perspektivischer und Seitenansicht sowie in mehreren weitergehenden Varianten;
- Figuren 8A und 8B: eine vierte Ausführungsform der ersten Grundform des Implantats;
- Figur 9: eine fünfte Ausführungsform der ersten Grundform des Implantats, eingesetzt in einen Schnittspalt;
- Figuren 10A und 10B: ein Implantat gemäß einer ersten Ausführungsform einer zweiten Grundform, eingesetzt in einen Schnittspalt in Draufsicht und Schnittansicht;
- Figur 11: Schnittansicht eines Implantats gemäß einer zweiten Ausführungsform der zweiten Grundform;
- Figuren 12A und 12B: ein Implantat gemäß einer ersten Ausführungsform einer dritten Grundform, eingesetzt in einen Schnittspalt in Draufsicht und Schnittansicht;
- Figuren 13A und 13B: eine zweite Ausführungsform eines Implantats gemäß der dritten Grundform;
- Figur 14: eine Variante der dritten Ausführungsform eines erfindungsgemäßen Implantats gemäß der ersten Grundform im implantierten Zustand;
- Figuren 15A und 15B: eine Variante der dritten Ausführungsform des Implantats gemäß der ersten Grundform im implantierten Zustand;
- Figuren 16A und 16B: zwei weitere Varianten der dritten Ausführungsform des Implantats gemäß der ersten Grundform in implantiertem Zustand;
- Figuren 17A und 17B: eine weitere Variante der dritten Ausführungsform des Implantats gemäß der ersten Grundform mit zugehörigem Halteelement;
- Figur 18: eine weitere Variante der dritten Ausführungsform des erfindungsgemäßen Implantats gemäß der ersten Grundform im implantierten Zustand in Schnittansicht;
- Figuren 19A und 19B: eine weitere Variante der dritten Ausführungsform des erfindungsgemäßen Implantats gemäß der ersten Grundform in Schnittansicht;
- Figuren 20A bis 20D: weitere Varianten der dritten Ausführungsform der ersten Grundform des Implantats in perspektivischer Darstellung;
- Figuren 21A bis 21C: verschiedene Detaildarstellungen einer weiteren Variante der dritten Ausführungsform des erfindungsgemäßen Implantats gemäß der ersten Grundform;
- Figuren 22A bis 22C: Schnittansichten einer Variante der dritten Ausführungsform der ersten Grundform des Implantats in unterschiedlichen Zuständen beim Implantieren;
- Figur 23A bis 23D: eine schematische Schnittansicht von zwei weiteren Varianten der dritten Ausführungsform der ersten Grundform des Implantats;
- Figuren 24A und 24D: zwei Varianten einer vierten Grundform des Implantats;
- Figur 25: eine fünfte Grundform des Implantats;
- Figur 26: einen Abschnitt einer Halswirbelsäule mit eingesetzten Implantaten der ersten Grundform;
- Figur 27: schematische Darstellung eines Implantatkörpers zur Parameterdefintion;
- Figur 28: Detailansicht eines implantierten Implantats der Figur 26 in teilweise aufgebrochener Darstellung des C6 Wirbels;
- Figuren 29A und 29B: eine erste Ausführungsform eines Mehrfachimplantat auf der Basis der ersten Grundform in perspektivischer Darstellung und Draufsicht im implantierten Zustand;
- Figur 30: eine zweite Ausführungsform eines Mehrfachimplantats auf der Basis der ersten Grundform im implantierten Zustand;
- Figur 31: eine dritte Ausführungsform eines Implantats auf der Basis der ersten Grundform in Seitenansicht; und
- Figur 32: eine vierte Ausführungsform eines Mehrfachimplantats auf der Basis der ersten Grundform in perspektivischer Darstellung.

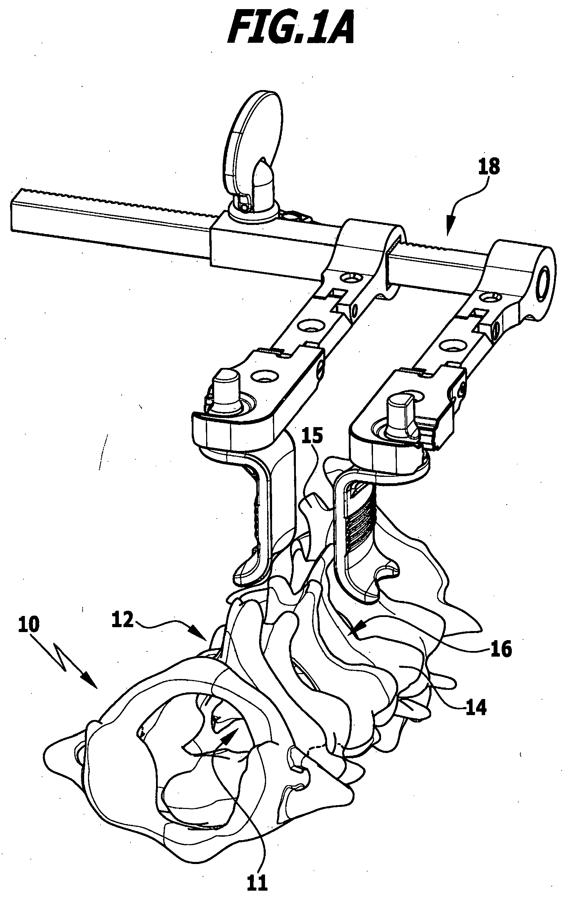

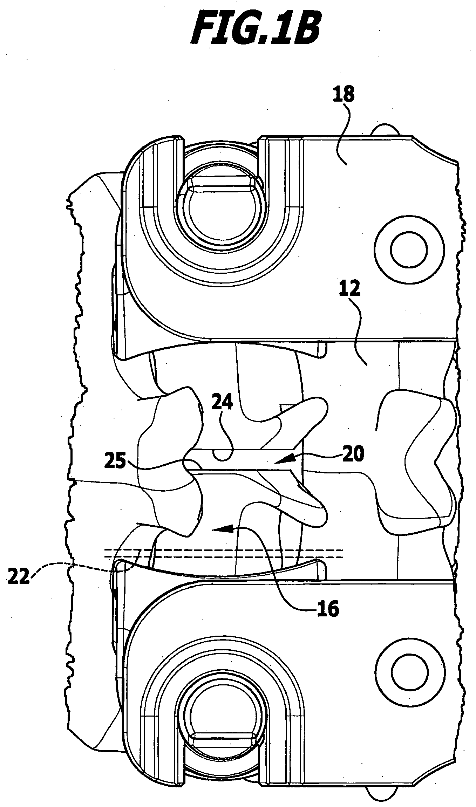

Figur 1A zeigt in schematischer Darstellung den Abschnitt C1 bis C7 einer Halswirbelsäule 10 mit einem Rückenmarkskanal 11 sowie einem über einem Wirbelbogen 12 eines Wirbels 14 positionierten Retraktor 18, der das umgebende (in der Figur 1 der Übersichtlichkeit halber weggelassene) Gewebe zurückhält, so dass der Bereich des Wirbels 14 dorsal zugänglich bleibt.

Der Zugang kann zu dem Wirbel 14 und dessen Wirbelbogen 12 ohne Ablösung von Muskelgewebe geschehen und so dessen Dornfortsatz 15 zugänglich gemacht werden, während der Zugang zur Lamina 16 eine Ablösung von Muskelgewebe notwendig macht.

In dem in Figur 1A gezeigten Zustand kann mit einem Instrument (nicht gezeigt) im Bereich der Lamina 16 oder im Bereich des Dornfortsatzes 15 ein einziger Schnittspalt 20 oder 22 erzeugt werden, der eine elastisch/plastische Aufweitung des Wirbelbogens 12 bzw. seiner durch den Schnittspalt 20 oder 22 getrennten Wirbelbogenabschnitte ermöglicht, so dass weitere Bereiche des Wirbels nicht freigelegt werden müssen.

Die Herstellung des Schnittspalts ist nicht auf eine bestimmte Verfahrensweise limitiert. So kann der Schnittspalt z.B. mit einem Ultraschall-Osteotom hergestellt werden, das eine besonders schonende Durchtrennung der Knochensubstanz bis zum Rückenmarkskanal 11 erlaubt. Eine Schädigung des Bindegewebes des Rückenmarks wird hier vermieden.

Alternativ kann mit schnell drehenden Bohrern gearbeitet werden, wobei die letzte Phase des Trennschnitts bis zum Rückenmark vorzugsweise mit einer Knochenstanze durchgeführt wird.

Eine weitere Alternative bietet die sogenannte T- oder Gigli-Säge, mit der ausgehend vom Rückenmarkskanal 11 der Schnittspalt hergestellt wird.

Figur 1B zeigt den Wirbel 14 mit im Dornfortsatz fertiggestelltem Schnittspalt 20. Der alternative Schnittspalt 22 in einer der Lamina des Wirbelbogens 12 ist in durchbrochener Darstellung angedeutet.

Im Folgenden soll die Erfindung anhand des in dem Dornfortsatz erzeugten Schnittspalts 20 beschrieben werden, wobei eine analoge Vorgehensweise für den Fachmann ersichtlich für ein Schnittspalt 22 in der Lamina gilt.

Der Schnittspalt 20 weist zwei in dem in Figur 1B gezeigten Zustand parallel angeordnete Schnittflächen 24 und 25 auf. Die in diesem Zustand vorhandene Spaltbreite hängt von der zur Erzeugung des Schnittspalts eingesetzten Technik ab und beträgt beispielsweise bei Verwendung eines Ultraschall-Ostetoms ca. 1 mm oder weniger. Bei schnelldrehenden Bohrern wird typischerweise eine Breite des Schnittspalts von ca. 2 mm bis 3 mm erhalten.

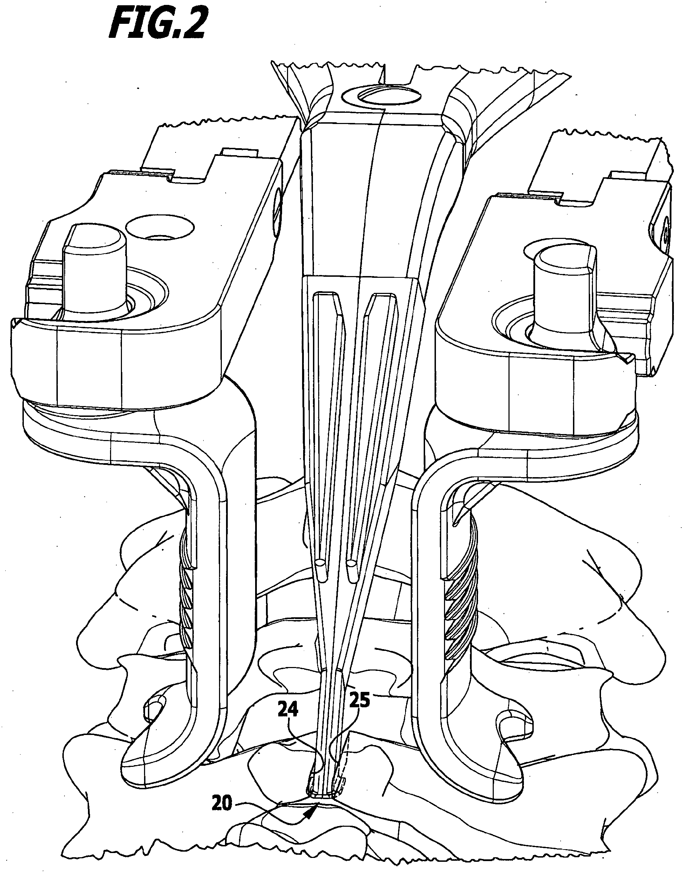

Zur elastisch/plastischen Aufweitung des Schnittspalts 20 wird ein Distraktionsinstrument 28 verwendet und dorsal in den Schnittspalt 20 eingeführt, wie dies in Figur 2 gezeigt ist. Vorzugsweise umfasst das Distraktionsinstrument eine Spaltbreitenanzeige und/oder eine Kraftmessvorrichtung (beide nicht gezeigt), so dass die erzielte Spaltbreite ablesbar ist bzw. die bei der Aufweitung aufgewendete Kraft dosiert eingesetzt werden kann. Weiter bevorzugt weist das Distraktionsinstrument 28 ein Rastelement (nicht gezeigt) auf, das eine einmal erreichte Aufweitposition des Distraktionsinstruments selbsttätig fixiert. Dieses Rastelement kann auch ein abgestuftes Aufweiten des Schnittspalts erleichtern.

Bevorzugt weist das Distraktionsinstrument 28 an seinem distalen Ende einen abgewinkelten Bereich auf, so dass auch beim Aufweiten des Schnittspalts 20 die Sicht erhalten bleibt und ein einfacher Zugang beim Einsetzen des Implantats in den Schnittspalt 20 gegeben ist.

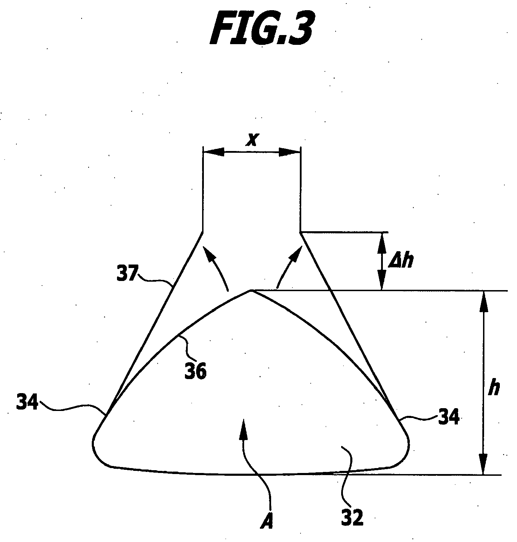

Die Ergebnisse bezüglich der Aufweitung des Wirbelkanaldurchmessers bzw. der Wirbelkanalfläche lassen sich an einem einfachen Modell anhand der Figur 3 erläutern.

Ausgangspunkt ist ein C6-Wirbel mit den in Figur 3 indizierten Parametern A = 150,65 mm² und einem Durchmesser h von 11,5 mm. Die Berechnungen für eine entsprechende Spaltbreite x basieren auf folgenden Annahmen:
- Die Gestalt des Spinalkanals im Wirbel kann durch ein gebogenes Dreieck 32 wie aus Figur 3 ersichtlich angenähert werden.
- Der Drehpunkt 34 der Wirbelbogenabschnitte liegt im Bereich der so genannten facet joints oder Füßchen.
- Die einzige elastisch/plastische Deformation der Abschnitte des Wirbelbogens 12 wird im Bereich der Lamina angenommen, wobei deren Bogenlänge der Einfachheit halber als konstant angenommen wurde und die Biegelinien 36, 37 als Kurven vereinfacht wurden.
- Der Wirbelkörper (nicht gezeigt) und die Anschlussstellen der Lamina an den Wirbelkörper (Füßchen) werden als starr angenommen.

In der Berechnung wurde die Öffnungsweite (Spaltbreite) x im Bereich von 6 bis 16 mm in 2 mm-Inkrementen erhöht. Die entsprechenden Werte für den Flächenzuwachs ΔA bzw. den Zuwachs an Durchmesser Δh sind in der folgenden Tabelle 1 aufgelistet.

**Tabelle 1**

| **Spaltbreite** | **Flächenzuwachs** | **Durchmesserzuwachs** |
|---|---|---|
| X | ΔA | Δh |
| 6 mm | 50,67 mm² | 3,79 mm |
| 8 mm | 63,30 mm² | 4,22 mm |
| 10 mm | 75,36 mm² | 4,53 mm |
| 12 mm | 86,72 mm² | 4,72 mm |
| 14 mm | 107,66 mm² | 4,99 mm |
| 16 mm | 126,84 mm² | 5,16 mm |

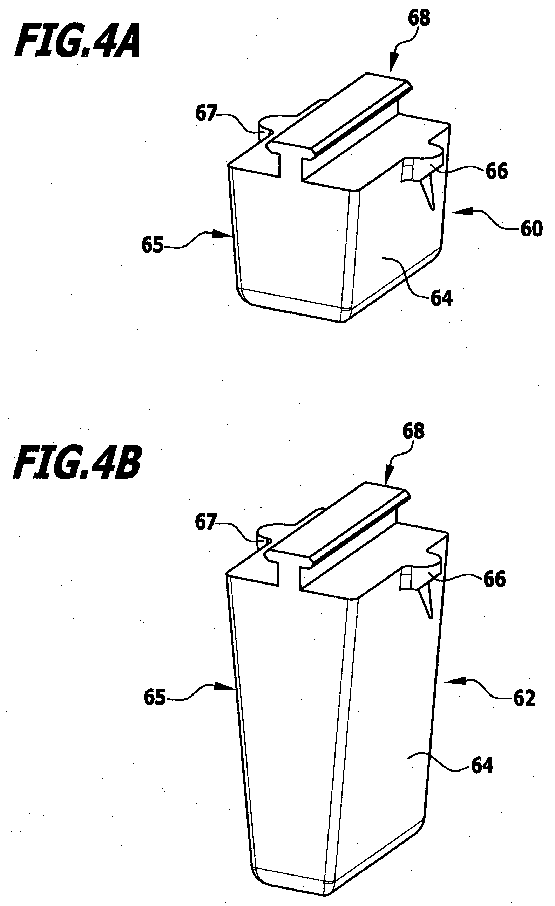

Ist der aufgeweitete Schnittspalt 20 bereit für das Einsetzen eines Implantats, so wird vorzugweise ein Implantatkörper 60 oder 62, wie beispielhaft in den Figuren 4A und 4B dargestellt, in den Schnittspalt 20 eingesetzt.

Die beiden Implantatkörper 60, 62 sind beide massiv ausgebildet und vorzugsweise aus einem Implantat-tauglichen Kunststoff, insbesondere PEEK, hergestellt. Die beiden Implantatkörper 60, 62 weisen gegeneinander geneigte Kontaktflächen 64, 65 auf, die mit den Schnittflächen des Schnittspalts im eingesetzten Zustand des Implantats in möglichst großflächigem Kontakt stehen. Während die Implantatkörper 60, 62 in Frontansicht keilförmig gestaltet sind, können sie in der Seitenansicht durchaus im Wesentlichen rechteckig ausgebildet sein.

An ihrem in den Figuren 4A und 4B oberen Ende, das im eingesetzten Zustand des Implantats dorsal liegt, weisen die Implantatkörper 60, 62 seitlich abstehende Vorsprünge 66, 67 auf, die mehrere Funktionen übernehmen können:
Zum Einen bewirken die Vorsprünge 66, 67, dass das Implantat nur bis zum Anschlag der Vorsprünge 66, 67 an der Knochensubstanz in den Schnittspalt eingeführt werden kann und auch in der nachöperativen Phase eine Verlagerung des Implantats in Richtung des Spinalkanals unterbunden wird.

Des Weiteren können die Vorsprünge 66, 67 im Formschluss mit einem Instrument oder mit entsprechenden Rücksprüngen in der Knochensubstanz eine Führung des Implantatkörpers beim Einsetzen des Implantats in den Schnittspalt bewirken und so eine präzise Platzierung unterstützen.

Schließlich vergrößern die Vorsprünge den dorsalen Bereich des Implantatkörpers und erleichtern somit die Aufnahme von Halteelementen wie z.B. Schrauben, Spikes etc., die der Fixierung des Implantats im aufgeweiteten Schnittspalt dienen.

Dorsal abstehend ist an den Implantatkörpern 60, 62 eine beidseits hinterschnittene Leiste 68 als Griffelement angeformt, die der besseren Handhabung beim Einsetzen und dem korrekten Positionieren des Implantats dient.

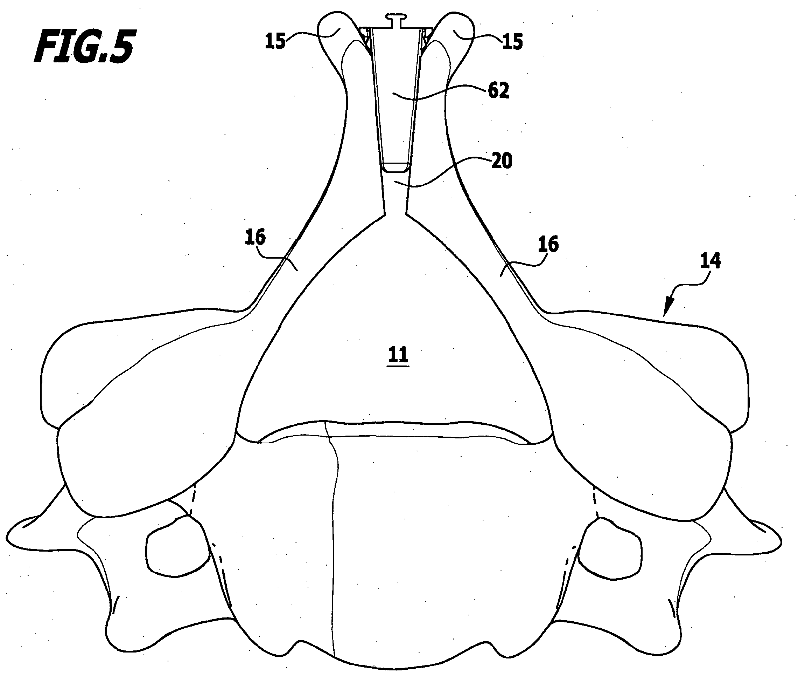

Der Implantatkörper 62 in seiner endgültigen Position im Schnittspalt 20 ist in Figur 5 dargestellt. Die Höhe des Implantatkörpers 62 ist in diesem Beispiel so gewählt, dass er im eingesetzten Zustand mit seinem ventral liegenden Ende nicht ganz an den Spinalkanal heranreicht, so dass dort ein zusätzliches Volumen für die Dekompression des Rückenmarks verbleibt. Der Winkel, in dem die Kontaktflächen keilförmig gegeneinander angeordnet sin, beträgt in diesem bevorzugten Beispiel ca. 10°.

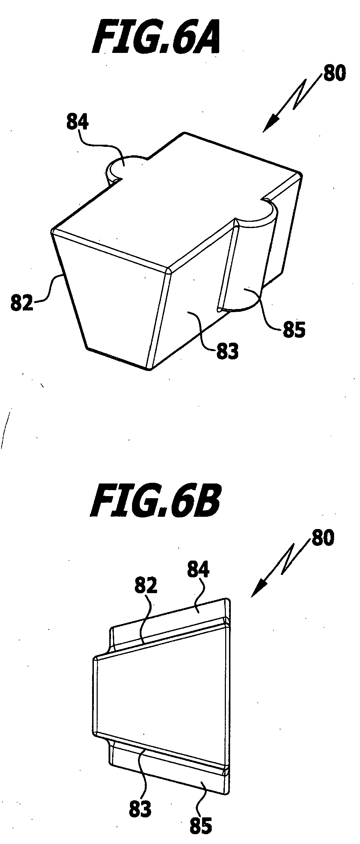

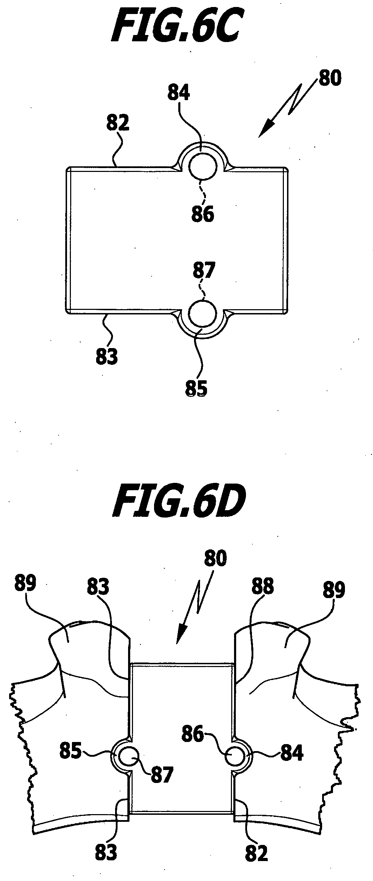

Figur 6 zeigt ein weiteres Ausführungsbeispiel eines Implantatkörpers 80 in perspektivischer Ansicht, Draufsicht, Frontansicht und im implantierten Zustand (in Draufsicht). Der Implantatkörper 80 weist wiederum Keilform mit gegeneinander geneigten Kontaktflächen 82, 83 auf, die im eingesetzten Zustand an den Schnittflächen eines Schnittspalts großflächig anliegen.

Bevorzugt weisen diese Implantatkörper 80 an ihren Kontaktflächen halbzylindrische Vorsprünge 84, 85 auf, die sich fast über die gesamte Höhe des Implantatkörpers 80 erstrecken. In den entsprechend vorbereiteten Schnittspalt sind deshalb komplementäre Nuten (hier nicht gezeigt) eingeformt, die beim Einsetzen das Implantat führen. Werden die Nuten nur zur Führung der Implantate verwendet, sind diese bevorzugt nicht bis zum Wirbelkanal durchgängig ausgebildet, so dass sich für das Einsetzen des Implantats 80 ein Anschlag im Knochenmaterial der Schnittflächen ergibt, an dem die Vorsprünge 84, 85 anliegen. Damit wird verhindert, dass die Implantatkörper 80 zu tief in den Schnittspalt eingesetzt werden können oder sich der Implantatkörper 80 zu einem späteren Zeitpunkt in Richtung des Spinalkanals verschiebt und dort zu einer Kompression führt.

In Figur 6C ist schematisch in durchbrochenen Linien eine weitere Ausgestaltung des Implantats 80 gezeigt. So können in dem Implantatkörper 83 Bohrungen 86, 87 vorgesehen werden, die dem Einsatz von Befestigungsmitteln wie z.B. Dübeln, Bolzen oder Schrauben dienen, wie dies im Folgenden noch in Einzelheiten dargestellt werden wird.

Die Vorsprünge 84, 85 erlauben dabei, die Bohrungsöffnungen im Implantatkörper 83 dorsal mit größerem Abstand voneinander enden zu lassen, wodurch eine höhere mechanische Belastbarkeit des Implantats resultiert und zudem der Einsatz der Befestigungsmittel vereinfacht wird.

Figur 6D zeigt das Implantat 80 in einem Schnittspalt 88 eingesetzt. Aus dieser Darstellung wird der Zweck der außermittigen Anordnung der Vorsprünge 84, 85 bezüglich der Sagittalrichtung des Implantats 80 deutlich: die korrespondierenden Rücksprünge im Schnittspalt des Wirbelbogens lassen sich zumindest zu eine erheblichen Teil außerhalb des gesplitteten Dornfortsatzes 89 anordnen, so dass nicht nur ein geringerer Eingriff in die Knochensubstanz beim Ausbilden der Rücksprünge resultiert, sondern auch das Einsetzen von Befestigungselementen in den Bohrungen 86, 87 einfacher zu bewerkstelligen ist.

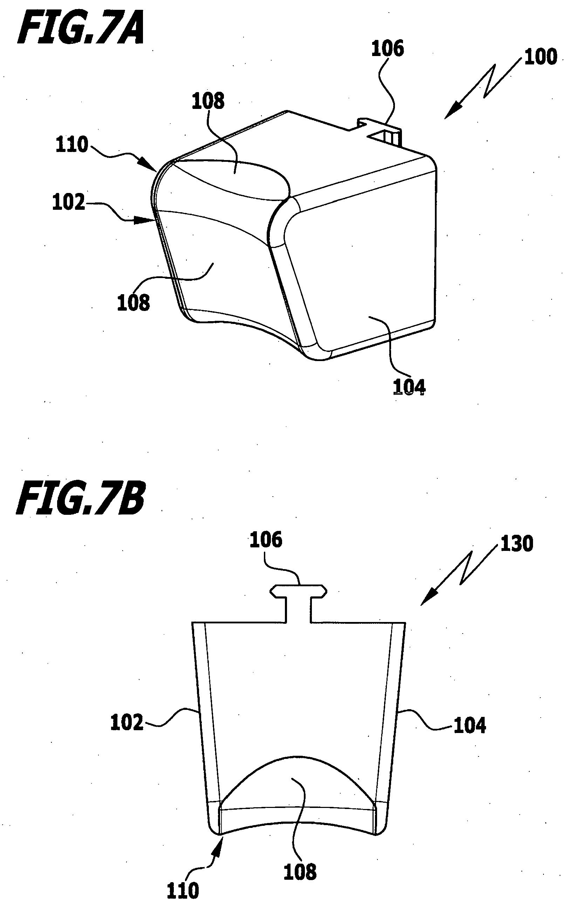

Die Figuren 7A und 7B zeigen im Einzelnen die detaillierte Ausgestaltung eines bevorzugten Implantats 100, der wie die zuvor besprochenen bevorzugten Implantatkörper keilförmig zueinander ausgerichtete Anlageflächen 102, 104 aufweist, die im eingesetzten Zustand im Schnittspalt zur Anlage an den Schnittflächen desselben kommen. An seinem dorsalen Ende ist eine Griffleiste 106 angeformt, mit der das Implantat präzise in den Schnittspalt eingesetzt werden kann.

Auf seiner ventralen Seite weist der Implantatkörper 100 eine Einbuchtung 108 auf, die sich um die ventral vorstehende Kante 110 erstreckt und eine weitere Vergrößerung des auf Seiten des Spinalkanals verfügbaren Raums und somit eine weitere Dekompression des Rückenmarks erlaubt. Mit dieser Maßnahme seitens des Implantatkörpers 100 wird ein zusätzlicher Raumgewinn für den Spinalkanal realisiert, der ansonsten nur durch ein wesentlich stärkeres Aufspreizen des Wirbelbogens erzielt werden könnte.

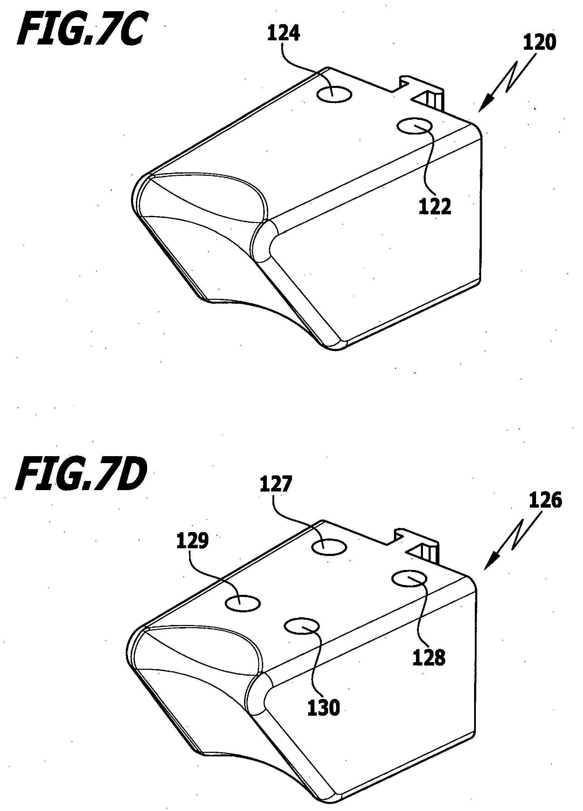

Die Figuren 7C bis 7F zeigen weitere Varianten des Implantats 100 der Figuren 7A und 7B.

Figur 7C zeigt ein Implantat 120 mit einer, vorzugsweise zwei Durchgangsöffnungen 122, 124, die parallel zur Sagittalebene angeordnet sind und die das Vernähen des Implantats mit dem Dornfortsatz erlauben.

Figur 7D zeigt ein Implantat 126 mit einem gegenüber dem Implantat der Figur 7C längeren Implantatkörper, der es erlaubt, zusätzliche Bohrungen vorzusehen, so dass eventuell abgebrochene Teile des Dornfortsatzes zusätzlich am Implantat fixiert werden können. Auch wenn hier insgesamt vier Bohrungen 127, 128, 129, 130 gezeigt sind, kann im einfacheren Fall je eine Bohrung oder Durchgangsöffnung distal und proximal bzw. ventral und dorsal ausreichen, um diesen Zusatznutzen zu gewährleisten.

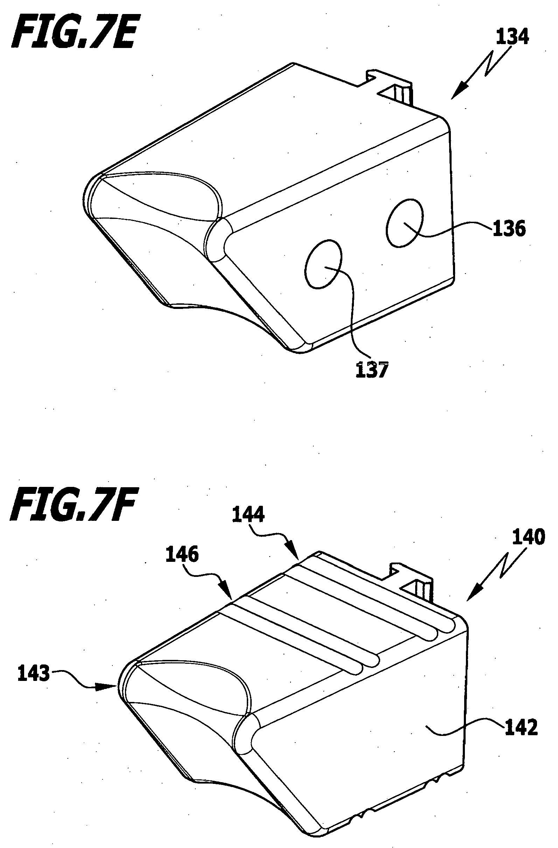

Figur 7E zeigt eine alternative Ausführungsform eines Implantats 134 zu Figur 7C, bei dem die Durchgangsöffnungen 136, 137 zum Vernähen des Implantats quer zur Sagittalebene angeordnet sind.

Eine weitere Alternative eines mittels Faden fixierbaren Implantats 140 zeigt Figur 7F. Hier weist das Implantat 140 an seinen quer zu den Kontaktflächen 142, 143 verlaufenden Oberflächen Rillen 144, 146 auf, in denen der Faden bzw. die Fäden beim Nähen geführt und gehalten werden, so dass sie nicht abrutschen können, wenn Querkräfte auf den Faden (nicht dargestellt) wirken.

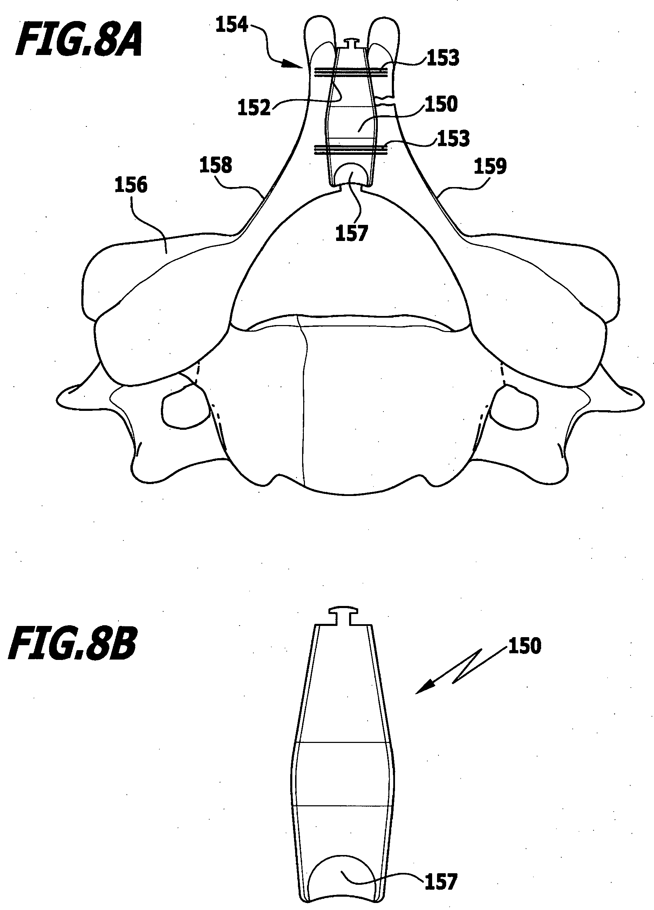

Die Figuren 8A und 8B zeigen eine weitere Variante des Implantats 100. Bei dieser Variante hat das Implantat 150 eine größere Höhe, die beispielsweise doppelt so groß sein kann wie die Höhe des Implantatkörpers 100, und nimmt bevorzugt die Form eines Doppelkeils an. Im eingesetzten Zustand in einem Schnittspalt 152 eines gesplitteten Dornfortsatzes 154 eines Wirbels 156, ist der ventral angeordnete Teil, der im Wesentlichen der Ausformung des Implantats 100 entspricht, wie er im Zusammenhang den Figuren 7A und 7B beschrieben wurde, im Bereich des Schnittspalts der Lamina positioniert, an den die Wirbelbogenabschnitte 158, 159 angrenzen. Der ventral angeordnete Teil weist entsprechend eine Einbuchtung 157 auf. Der dorsal angeordnete Teil ist zwischen den Teilen des gesplitteten Dornfortsatzes 154 angeordnet.

Diese Variante des Implantats 150 hat den Vorteil, dass beispielsweise ein frakturiertes Stück des Dornfortsatzes 154 wieder angebunden werden kann, so dass ein erneutes Zusammenwachsen des frakturierten Stücks möglich ist. Auch unter kosmetischen Gesichtspunkten wird hier ein besseres Operationsergebnis erzielt.

Figur 8A zeigt das Implantat 150 schematisch über Fäden 153 am Dornfortsatz bzw. der Lamina im Schnittspalt 152 fixiert.

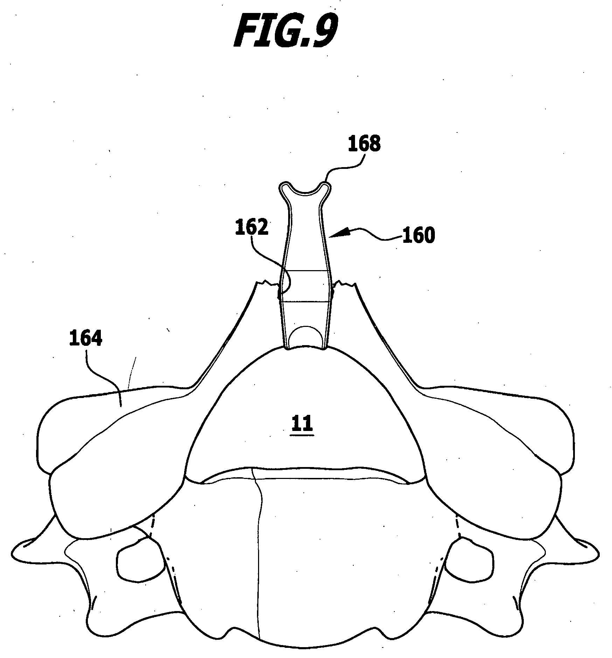

Unter ähnlichen Gesichtspunkten ist das Implantat 160 der Figur 9 entwickelt, das bei Operationen zum Einsatz kommen kann, bei denen der Dornfortsatz teilweise entfernt werden muss. Figur 9 zeigt das Implantat 160 in den Schnittspalt 162 eines Wirbels 164 eingesetzt. Das posteriore Ende 168 (dorsal) des Implantatkörpers 160 bildet die voroperative Form des Dornfortsatzes nach und erzielt so trotz der entfernten Teile des Dornfortsatzes ein kosmetisch zufrieden stellendes Ergebnis nach der Operation. Des Weiteren bietet diese Ausführungsform des Implantats die Möglichkeit, abgelöste Muskulatur wieder anzubringen.

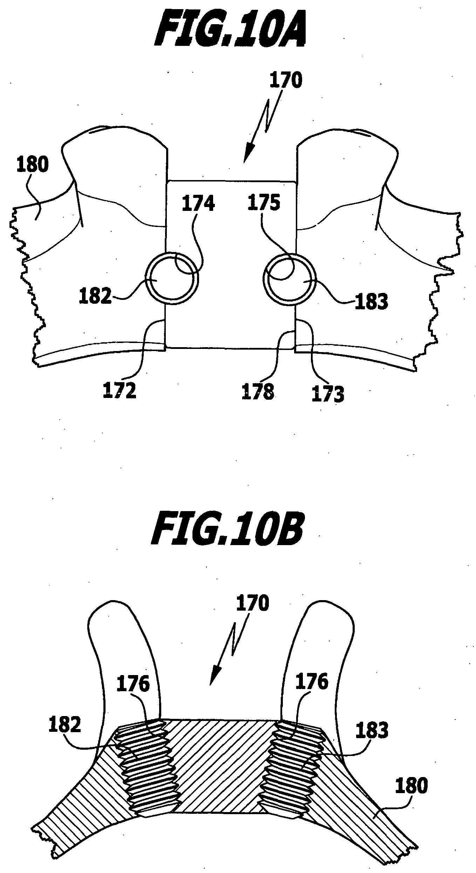

Die Figuren 10A und 10B zeigen ein Implantat 170, das sich von der bisher beschriebenen Grundform eines Implantats dadurch abhebt, dass an den keilförmig angeordneten Kontaktflächen 172, 173 Nuten-artige Rücksprünge 174, 175 ausgebildet sind, die im Wesentlichen bei der Ausführungsform der Figur 10 halbzylindrisch ausgestaltet sind und in die ein Innengewinde 176 eingeschnitten ist.

Der Schnittspalt 178 des Wirbelbogens 180 wird mit komplementären Rücksprüngen von halbzylindrischer Form an seinen Schnittflächen ausgebildet, so dass im eingesetzten Zustand des Implantats 170 im Schnittspalt 178 zunächst im Wesentlichen zylindrische Öffnungen vorliegen.

Danach werden Schraubbolzen 182, 183 in diese zylindrischen Öffnungen eingeschraubt, wobei die Schraubbolzen von dem im Implantat 170 bereits vorhandenen Innengewinde 176 geführt werden. Auf Seiten der Rücksprünge der Schnittflächen des Schnittspalts 178 schneiden sich die Schraubbolzen 182, 183 das Gewinde beim Einschrauben selbst ein, mit dem Vorteil, dass gleichzeitig mit dem Formschluss des Gewindes auch noch ein Kraftschluss erzielt wird, der die Schraubbolzen 182, 183 im fertig eingeschraubten Zustand auch langfristig hält.

Das Material der Schraubbolzen 182, 183 ist vorzugsweise PEEK wie auch der Körper des Implantats 170 vorzugsweise aus PEEK hergestellt wird, so dass bei Folgeuntersuchungen mittels MRT eine ungestörte Beobachtung des erweiterten Spinalkanals möglich ist.

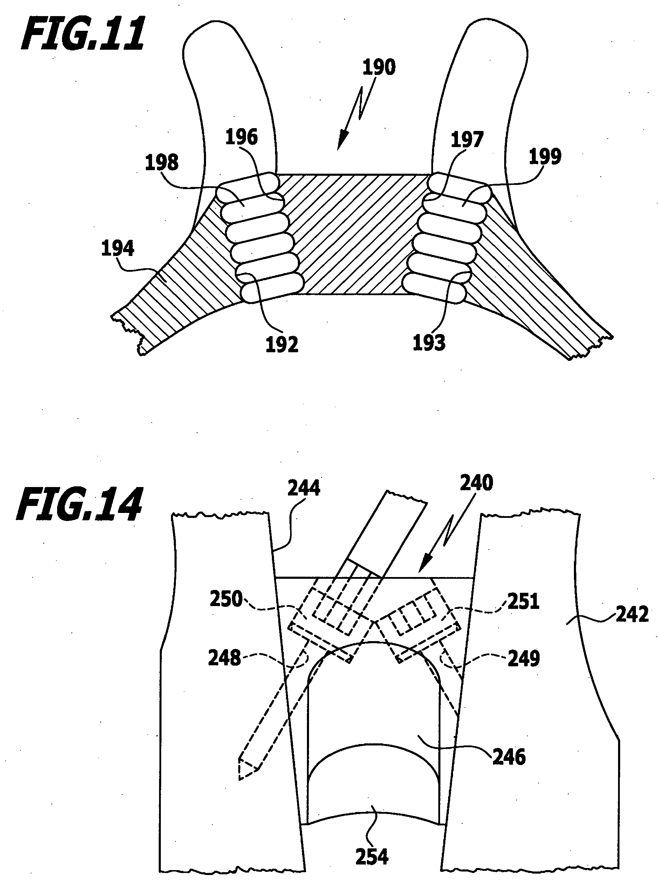

Figur 11 zeigt eine zweite Ausführungsform der zweiten Grundform in Form des Implantats 190, ebenfalls in einen Schnittspalt 192 eines Wirbelbogens 194 eingesetzt. Das Implantat 190 weist, wie im Zusammenhang mit der Figur 10A und 10B beschrieben, an seinen Kontaktflächen halbzylindrische Rücksprünge auf, so dass sich im eingesetzten Zustand des Implantats 190 im Wirbelbogen 174 wiederum im Wesentlichen zylindrische Hohlräume ergeben. In diese Hohlräume, gebildet durch die Nuten 192, 196 sowie 193, 197 werden dann Dübelelemente 198, 199 eingesetzt, die aus einem Material hergestellt sind, das durch Einbringen von Energie, z.B. Ultraschall, Wärme, UV-Licht, HF, etc., kurzzeitig plastifiziert werden und sich so den Gegebenheiten der Oberflächen des Schnittspalts des Wirbelbogens 194 einerseits und den Oberflächen der Rücksprünge 196, 197 des Implantats 190 anpassen kann. Dadurch wird eine formschlüssige Verbindung erzielt.

Die Rücksprünge 192, 193 in den Schnittflächen des Schnittspalts des Wirbelbogens 194 sind diesbezüglich vorzugsweise mit Rillen oder einem Gewinde versehen, so dass ein stärkerer Formschluss erzielt wird. Genau dasselbe gilt für die Rücksprünge 196, 197 auf Seiten des Implantats 190.

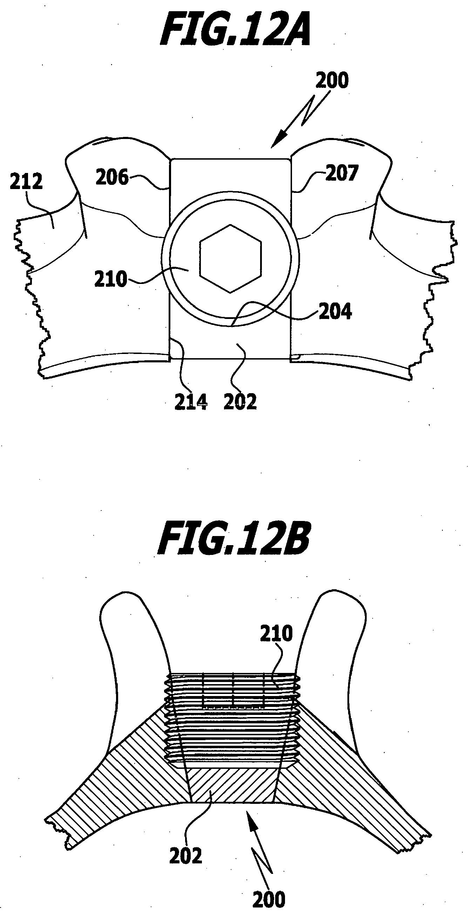

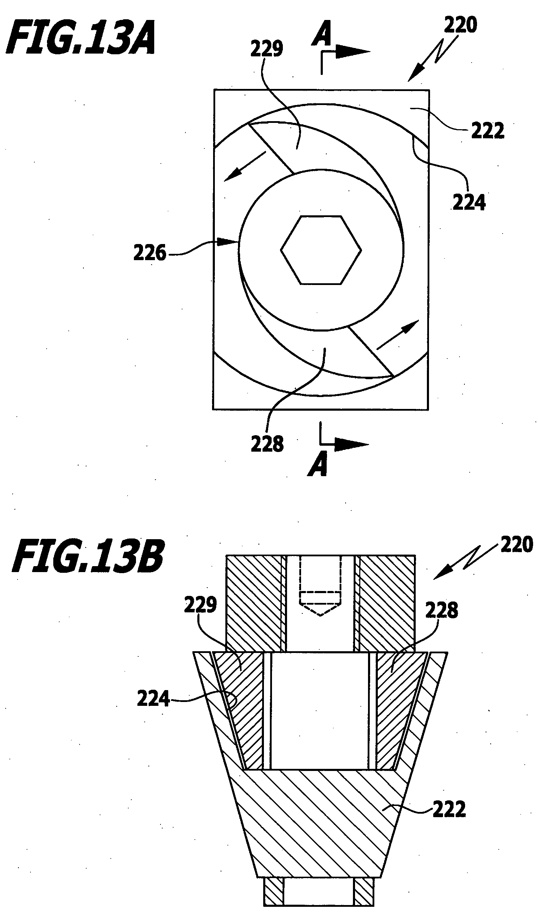

Die Figuren 12 und 13 zeigen eine dritte Grundform des Implantats, zunächst die Figuren 12A und 12B in Form eines Implantats 200.

Hier weist der Implantatkörper 202 eine Ausnehmung 204 auf, die im dorsalen Endbereich des Implantatkörpers 202 sacklochartig angeordnet ist und an ihren Wänden vorzugsweise ein Innengewinde ausgebildet aufweist.

Der Durchmesser des Sackloches 204 ist größer als der Abstand der Kontaktflächen 206, 207 des Implantatkörpers 202 an seinem dorsalen Endbereich. Dies erlaubt das Einbringen eines Verriegelungselements 210, das beispielsweise die Form einer Knochenschraube aufweisen kann. Wenn das Implantat . 200 in einem Wirbelbogen 212 bzw. dessen Schnittspalt 214 eingesetzt ist, kann die zentrale Knochenschraube 210 mit einem Durchmesser größer als der Abstand der Kontaktflächen 206, 207 in den Implantatkörper 202 eingeschraubt werden, wobei sich der Außenumfang bzw. das dort sich befindliche Gewinde des Verriegelungselements 210 in die Knochensubstanz des Wirbelbogens 212 einschneidet und so für einen Formschluss zwischen dem Implantat 200 und der Knochensubstanz des Wirbelbogens 212 sorgt. Ist der Durchmesser des Verriegelungselements 210 deutlich größer als der Abstand der Kontaktflächen 206, 207 des Implantatkörpers 202 an dessen dorsalem Endbereich, wie dies in den Figuren 12A und 12B dargestellt ist, so empfiehlt es sich, den Schnittspalt 214 vor dem Einsetzen des Implantats 200 an seinen Schnittflächen mit Rücksprüngen vorzubereiten, so dass eine zu große Verpressung der Knochensubstanz oder eine zu große Krafteinwirkung durch das Eindrehen des Verriegelungselements 210 beim Fixieren des Implantats 200 im Schnittspalt 214 vermieden wird und die Kontaktflächen 206, 207 auch weiterhin an den Schnittflächen des Schnittspalts anliegen.

Figur 13 zeigt ein Implantat 220, das auf einem ähnlichen Grundprinzip beruht wie das Implantat 200 der Figuren 12A und 12B. Das Implantat 220 weist in seinem Implantatkörper 222 ein Sackloch 224 auf, in welches wiederum vorzugsweise ein Innengewinde eingeformt ist.

In dem Sackloch 224 ist ein Verankerungselement 226 drehbar gehalten, welches am Außenumfang seines zylindrischen Grundkörpers zwei Flügel 228, 229 aufweist, die sich bei einer Drehbewegung des Verriegelungselements 226 in benachbart zu dem Implantatkörper 222 angeordnete Schnittflächen eines Schnittspalts eines Wirbelbogens einpressen. Die Figur 13B zeigt das Implantat in Schnittdarstellung längs Linie A-A der Figur 13A.

Figur 14 zeigt ein erfindungsgemäßes Implantat 240, eingesetzt in den Schnittspalt 244 eines Wirbelbogens 242 mit Dornfortsatz. Der Implantatkörper entspricht im Wesentlichen in seiner Ausgestaltung dem Implantatkörper 100 der Figuren 7A und 7B, wobei der Implantatkörper 246 zwei im spitzen Winkel zu den Kontaktflächen des Implantats 240 angeordnete Durchgangsbohrungen 248, 249 aufweist, die sich vom dorsalen Endbereich des Implantats aus bis zur jeweiligen Kontaktfläche erstrecken.

In diese Durchgangsbohrungen 248, 249 sind Stifte 250, 251 eingesetzt, die aus der Bohrung 248 bzw. 249 an den Kontaktflächen des Implantatkörpers 246 austreten und an dieser Stelle dann in die umgebende Knochensubstanz des Wirbelbogens 242 eindringen.

Es können Stifte mit einem Gewindeabschnitt an der Spitze verwendet werden, so dass sich der Stift beim Eindrehen in das Knochenmaterial selber einschneidet oder aber glatte Stifte verwendet werden, die durch leichte Schläge in das Knochenmaterial eingepresst werden. Bevorzugt wird beim Einbringen der Stifte jedoch das Implantat in seiner korrekten Position im Schnittspalt 244 gehalten, damit keine Verschiebung in ventraler Richtung, d.h. in Richtung zum Spinalkanal, erfolgen kann.

Das Sichern der Stifte 250, 251 in den Bohrungen 248, 249 kann durch an sich bekannte Sicherungsmittel erfolgen, wie sie z.B. von Pedikel-Schraubsystemen oder cervikalen Plattensystemen bekannt sind. Vorzugsweise sind, wie in Figur 14 abgebildet, die Durchgangsöffnungen 248, 249 mit einem dorsal erweiterten Abschnitt ausgebildet, so dass sich beim Übergang zu dem ventralen Abschnitt der Durchgangsöffnungen 248, 249 ein Anschlag ausbildet an dem dann ein Kopf des Stiftes mit größerem Durchmesser anlegt, so dass auch die endgültige Position des Stifts im Vorhinein festgelegt werden kann. Auf seiner ventralen oder dem Spinalkanal benachbarten Seite weist der Implantatkörper 240 wie der Implantatkörper aus den Figuren 7A und 7B eine Einbuchtung 254 auf, um so zusätzliches Volumen für den Spinalkanal zu schaffen.

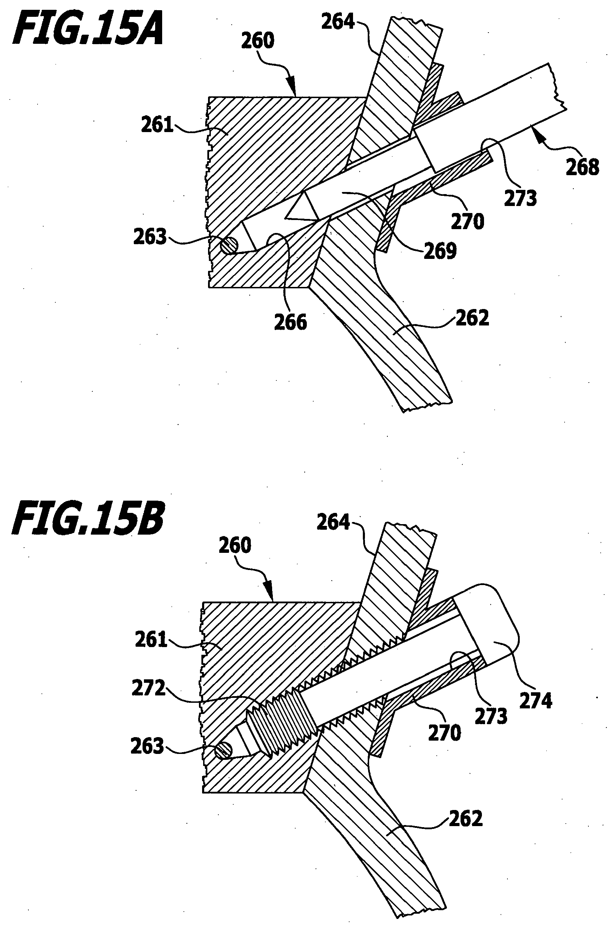

Im Figur 15 ist schematisch ein weiteres Implantat 260 der vorliegenden Erfindung gezeigt bei dem im Vergleich zum Implantat 240 der Figur 14 ein umgekehrtes Prinzip zur Befestigung im Wirbelbogen bzw. dessen Dornfortsatz 262 und dessen Schnittspalt 264 angewandt wird.

Zunächst zeigt Figur 15A das Implantat 260 im Schnittspalt 264 eingesetzt. Am Dornfortsatz 262 wurde partiell die Muskulatur abgelöst.

Das Implantat 260 enthält im Implantatkörper 261 integriert einen Tantal-Marker in Form einer Kugel 263, die im Röntgenbild den Endpunkt einer von außen eingebrachten Bohrung 266 anzeigt.

An der Außenfläche des Dornfortsatzes 262 wird ein Bohrwerkzeug 268 mit einem gestuften Bohrer 269 sowie einem Auflageteller 270 angesetzt und unter Röntgensicht die Bohrung im Dornfortsatz bzw. der Lamina erzeugt.

Anschließend kann das Bohrwerkzeug entfernt werden. Wie in Figur 15B gezeigt kann der Auflageteller 270 außen am Dornfortsatz 262 verbleiben, so dass dessen Öffnung 273 weiterhin mit der Bohrung 266 fluchtet.

Durch die Öffnung 273 des Auflagetellers 270 wird ein Befestigungselement 274, insbesondere eine Schraube durchgesteckt und mit dem Implantatkörper 261 verschraubt. Der Gewindeabschnitt 272 der Schraube 274 weist einen Hinterschnitt auf, der sich sein Gegengewinde im Implantatkörper 261 selbsttätig schneidet.

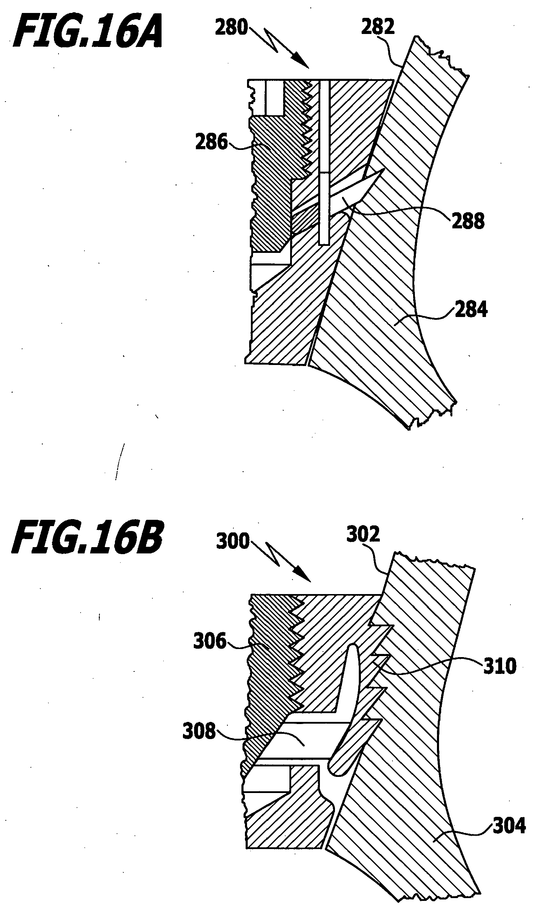

Die Figur 16 zeigt in den beiden Varianten A und B weitere Implantate 280 bzw. 300 eingesetzt in einem Schnittspalt 282 bzw. 302 eines Wirbelbogens 284 bzw. 304. Gemeinsam ist beiden Implantaten 280, 300 der generelle Mechanismus zur Befestigung des Implantats 280 bzw. 300 in dem jeweiligen Schnittspalt 282 bzw. 302, bei dem über eine Kulissenführung Verankerungselemente in die umgebende Knochensubstanz des Schnittspalts 282 bzw. 302 eingetrieben werden.

Zu diesem Zweck weist das Implantat 280 der Figur 16A ein Betätigungselement 286 auf, welches sich zu seiner ventral liegenden Spitze hin verjüngt und so eine Kulisse bildet um ein Verriegelungselement 288, das verschieblich in einer seitlichen Öffnung des Implantatkörpers 280 gelagert ist nach außen, d.h. über die Kontaktfläche des Implantats 280 hinaus zu treiben und so in die umgebende Knochensubstanz des Wirbelbogens 284 eindringen zu lassen. Vorzugsweise ist das Verriegelungselement 288 mit einer scharfen Spitze versehen, so dass es nur eine geringe Kraftaufwendung bedarf, um es in die spongiöse Knochensubstanz des Schnittspalts 282 hineinzutreiben.

Eine alternative Lösung hierfür ist in der Ausführungsform der Figur 16B gewählt bei der ebenfalls ein Betätigungselement 306 mit einer konisch zulaufenden Spitze eine Kulisse bildet, über die mittels eines Kraftübertragungsbolzens 308 ein beweglich an der Kontaktfläche des Implantats 300 gehaltenes Verankerungselement in die umgebende spongiöse Knochensubstanz eingetrieben wird. Das Verankerungselement 310 kann vorzugsweise einstückig mit dem Implantat 300 ausgebildet sein und insbesondere bei einer Fertigung des Implantats aus PEEK-Material aufgrund dessen Elastizität ausreichend beweglich am Implantatkörper selbst gehalten sein.

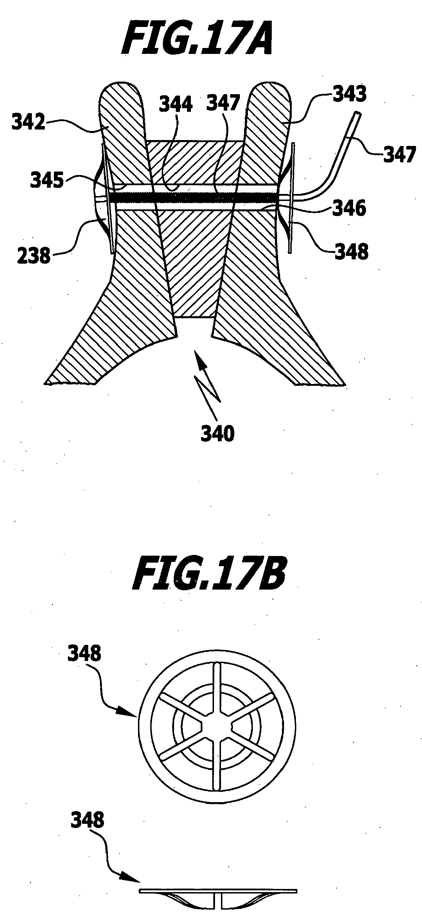

Die Figur 17A zeigt eine weitere Variante eines Implantates 340 in der Einbausituation zwischen den Hälften 342, 343 eines gesplitteten Dornfortsatzes.

Der Implantatkörper 340 weist eine quer zur Implantatlängsrichtung oder -tiefe angeordnete Bohrung 344 auf, welche mit entsprechenden Bohrungen 345, 346 oder Ausnehmungen im Dornfortsatz 342,343 fluchtet.

Im eingesetzten Zustand des Implantatkörpers 340 im Dornfortsatz 342, 343 wird dann beispielsweise ein Drahtsegment 347 durch die Bohrung 344 sowie die Ausnehmung 345, 346 hindurch gesteckt und an seinem freien Ende mit einem Halteelement 348 fixiert. Das zweite freie Ende des Drahtsegments 347 wird ebenfalls mit einem Halteelement 348 bestückt, wie es beispielhaft in der Figur 17B in vergrößerter Darstellung in Draufsicht und Seitenansicht wiedergegeben ist.

Bevorzugt weist das Drahtsegment 347 eine Struktur auf, die es erlaubt, die Halteelemente 348 ohne spezielles Werkzeug darauf rastend zu befestigen, so dass nach Einsetzen des Drahtsegments 347 und der darauf fixierten Halteelemente 348 das Drahtsegment 347 einfach gespannt und die Halteelemente 348 zur Anlage an den Außenflächen der Dornfortsatzteile 342, 343 gebracht werden können und so den Implantatkörper 340 im Schnittspalt sichern.

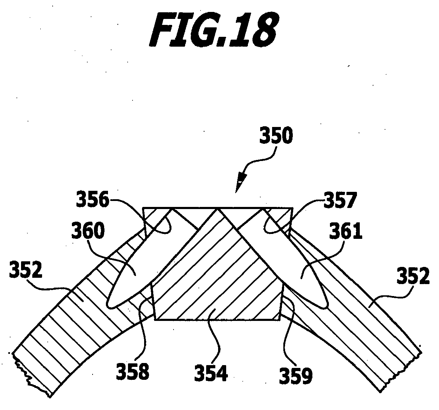

Figur 18 zeigt eine Schnittdarstellung der Einbausituation eines erfindungsgemäßen Implantats 350 im Schnittspalt eines Wirbelbogens 352, wobei in dem Implantatkörper 354 zwei Bohrungen 356, 357 vorgesehen sind, die in einem spitzen Winkel zu den Kontaktflächen des Implantatkörpers 354 verlaufen, mit dem dieser an den Schnittflächen des Schnittspalts des Wirbelbogens 352 anliegt. Die Bohrungen 356, 357 verlaufen von der dorsalen Oberfläche des Implantatkörpers 354 und treten aus den Kontaktflächen 358, 359 aus.

Wird das Implantat 350 im Wirbelbogen 352 fixiert, werden in die Bohrungen 356, 357 spitz zulaufende Bolzen 360, 361 eingesetzt, die mit ihrer Spitze im eingebauten Zustand über die Kontaktflächen 358, 359 austreten und dabei in die umgebende Knochensubstanz des Wirbelbogens 352 eindringen.

Die Bolzen 360, 361 können verschieden ausgeführt werden. Gemäß einer Ausführungsform können die Bolzen 360, 361 aus einem plastifizierbaren Material bestehen und werden im eingesetzten Zustand kurzzeitig plastifiziert, so dass sich eine formschlüssige Verbindung zwischen dem Material der Bolzen 360, 361 und der sie an ihren Spitzen umgebenden Knochensubstanz des Wirbelbogens 352 ergibt.

Gemäß einer anderen Ausführungsform sind die Bolzen als so genannte Spikes ausgeführt und halten in dem Implantatkörper 354 sowie den angrenzenden Anteilen der Knochensubstanz des Wirbelbogens 352 im Wesentlichen kraftschlüssig.

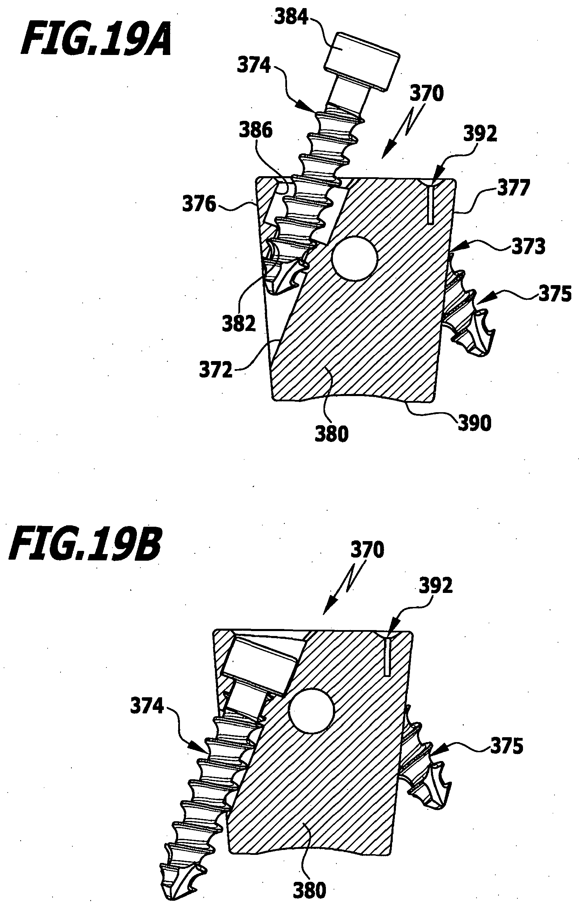

Gemäß einer weiteren in den Figuren 19A und 19B gezeigten Variante eines erfindungsgemäßen Implantats 370 sind die Bolzen als Schraubbolzen 374, 375 ausgeführt, die mit ihrer Spitze in die an den Kontaktflächen 376, 377 des Implantats anliegende Knochensubstanz des Wirbelbogens (nicht dargestellt) eingeschraubt werden.

Die Abbildung A der Figur 19 zeigt eine Schnittansicht durch den Implantatkörper 380, der - ähnlich wie dies für Figur 18 schon beschrieben wurde - zwei gegenüber den Kontaktflächen des Implantatkörpers 380 spitzwinklig verlaufende Durchgangsöffnungen 372, 373 aufweist. Die beiden Durchgangsöffnungen 372, 373 sind hier, anders als beim Implantat 350 der Figur 18, gestaffelt angeordnet, so dass in den Schnittdarstellungen A und B der Figur 19 nur der in der Zeichnung vorn liegende Durchgangskanal 372 vollständig sichtbar ist. In beiden Durchgangsöffnungen 372, 373 sind Schraubbolzen 374, 375 angeordnet, von denen der Schraubbolzen 375 bereits vollständig in die Durchgangsöffnung 373 eingeschraubt ist und mit seinem spitzen Ende über die Kontaktfläche hinaus in die Knochensubstanz eines Wirbelbogens (hier nicht dargestellt) eindringt.

Der Schraubbolzen 374 ist in der Darstellung A der Figur 19 noch in seiner Ausgangsstellung gezeigt, in der er von einem kurzen Gewindeabschnitt 382, der einen Teil der Durchgangsöffnung 372 bildet, formschlüssig gehalten wird.

Nachdem der Implantatkörper 380 in einen Schnittspalt eines Wirbelbogens eingesetzt ist, werden die beiden Schraubbolzen 374, 375 betätigt und so das Implantat 370 mit der umliegenden Knochensubstanz verschraubt. Der Gewindeabschnitt 382 führt dabei die Schraubbolzen zusammen mit den restlichen Teilen der Durchgangsöffnung 372, so dass sie in einer vorgegebenen Orientierung in die umgebende Knochensubstanz eindringen können.

Die Durchgangsöffnungen 372, 373 weisen an ihren dorsal liegenden Öffnungen an der Oberfläche des Implantats 370 einen größeren Durchmesser auf, so dass die Schraubbolzen 374, 375 im eingeschraubten Zustand mit ihrem Schraubenkopf 384 ganz innerhalb des Körpers des Implantats 370 aufgenommen werden können. An diesen Bereich 386 mit erweitertem Durchmesser der Durchgangsöffnung 372, 373 schließt sich der zuvor schon erwähnte Abschnitt mit einem Innengewinde 382 an.

Die Schraubbolzen 374, 375 werden vorzugsweise so gestaltet, dass an ihrem dem Schraubenkopf 384 benachbarten Bereich ein so genannter Freilauf 388 vorgesehen ist, der lang genug ist, um das Innengewinde 382 der Durchgangsöffnung 372 zu durchsetzen, so dass bei vollständig eingeschraubten Schraubbolzen 374, 375 das Gewinde der Schraubbolzen außer Eingriff mit dem Innengewinde 382 gerät und dadurch der Implantatkörper 380 durch Anziehen der Schraubbolzen 374, 375 zu einer engen Anlage mit seinen Kontaktflächen an die Schnittflächen der Knochensubstanz gebracht werden kann.

Die Länge der Schraubbolzen 374, 375 ist so bemessen, dass sie auch nach vollständigem Einschrauben in den Implantatkörper 380 nicht so weit aus dem Implantatkörper herausstehen, dass sie in den Spinalkanal eindringen könnten. Vorzugsweise ist deshalb die Spitze der Schraubbolzen 374, 375 in vollständig eingeschraubtem Zustand hinter der ventralen Vorderkante 390 des Implantats 370 zurückbleibend positioniert.

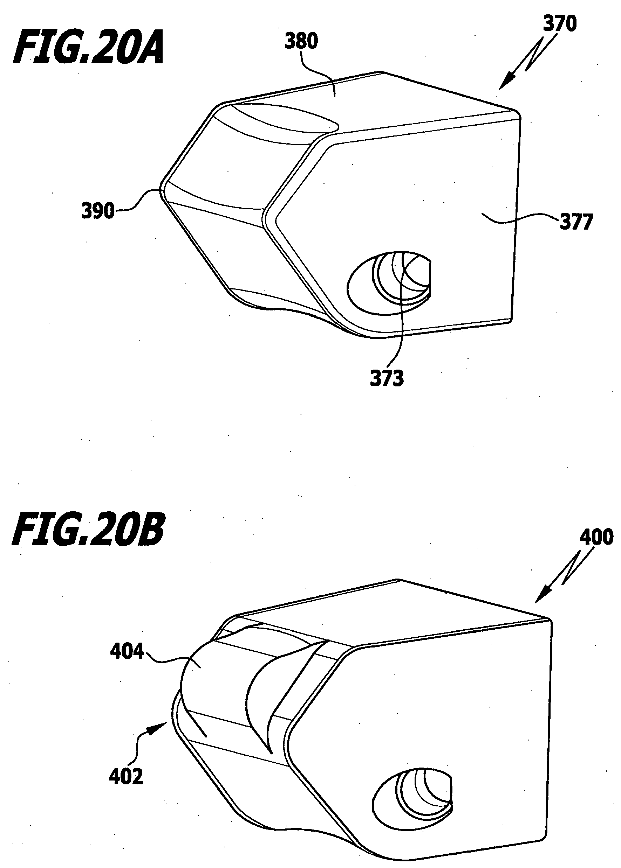

Die Figur 20A zeigt zum Vergleich mit den in den Figuren 20B bis D dargestellten Varianten nochmals das Implantat 370 der Figur 19, diesmal in perspektivischer Darstellung den ventralen bzw. distalen Endbereich 390 (Vorderkante) des Implantats, das mit einer Einbuchtung versehen ist, so dass zusätzliches Volumen benachbart zum Spinalkanal geschaffen wird.

In der Alternative der Figur 20B weist das Implantat 400 eine im Wesentlichen ähnliche Struktur zu dem Implantat 370 auf, mit dem Unterschied, dass am ventralen Endbereich 402 eine Nase 404 angeformt ist, welche beim Einführen des Implantats in den Schnittspalt des Wirbelbogens zwischen die Branchen einer Distraktionszange eingreifen kann, so dass das Implantat 400 geführt, insbesondere zentriert in den Schnittspalt des Wirbelbogens eingeführt werden kann.

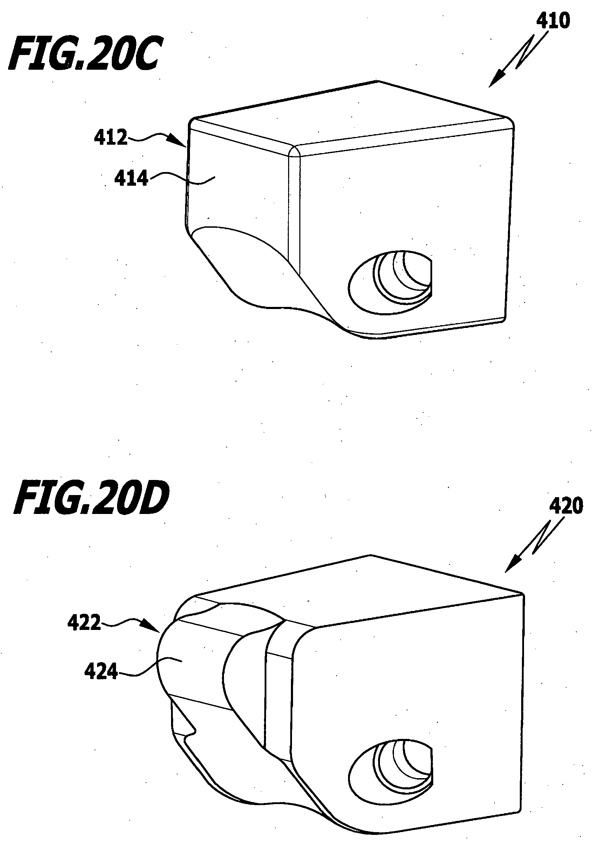

Die Figur 20C zeigt ein Implantat 410 mit einer ebenen Ausgestaltung des ventralen Endbereichs 412, der damit eine Anschlagfläche 414 bildet, die einen großflächigen Kontakt mit der Distraktionszange bzw. deren Anschlagselementen im fertig eingesetzten Zustand im Schnittspalt ermöglicht.

Eine weitere Variante eines Implantats ist in Figur 20D gezeigt. Das dort dargestellte Implantat 420 weist einen ventralen Endbereich 422 auf, bei dem, ähnlich wie in der Ausführungsform der Figur 20B, eine Nase 422 vorsteht, die ebenfalls eine Führung des Implantats 420 beim Einsetzen gewährleistet, indem es damit zwischen die Branchen eines Distraktionsinstruments eingreifen kann.

Um die erfindungsgemäßen Implantate besser handhaben zu können, werden diese bevorzugt an einem Halte- oder Einführinstrument befestigt, und bevorzugte Implantate weisen zur Verbindung mit dem Halte- bzw. Einführinstrument entsprechende Merkmale auf, beispielsweise eine Bohrung in der dorsal gelegenen Endfläche, wie dies in der Figur 19A und 19B beispielhaft als Bohrung 392 dargestellt ist. In dieser lässt sich ein Halte- bzw. Einführinstrument parallel zur Längsrichtung des Implantats fixieren.

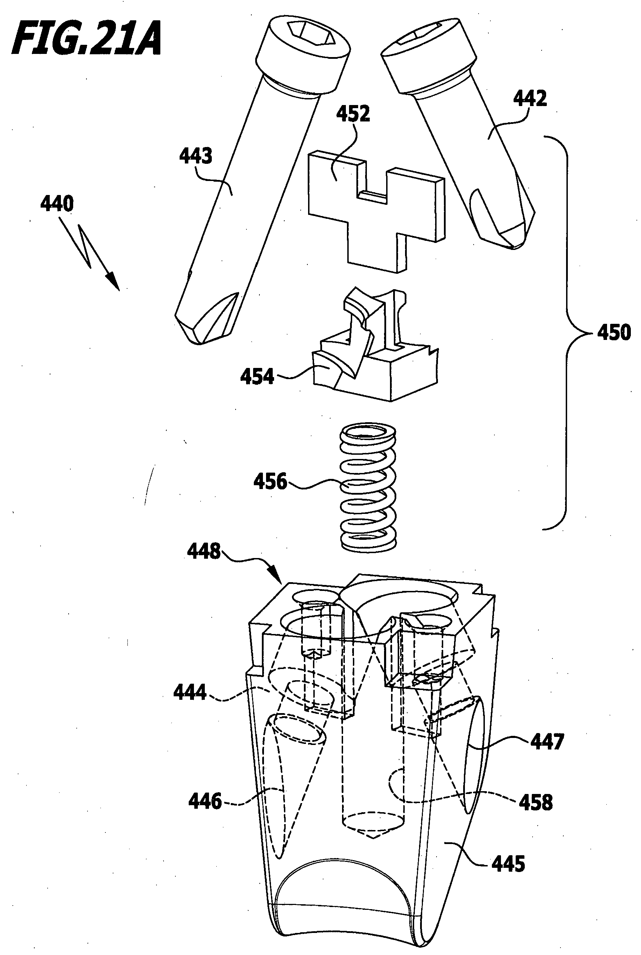

Ein weiterer Aspekt der erfindungsgemäßen Implantate soll im Zusammenhang mit den Figuren 21A bis C noch näher beleuchtet werden.

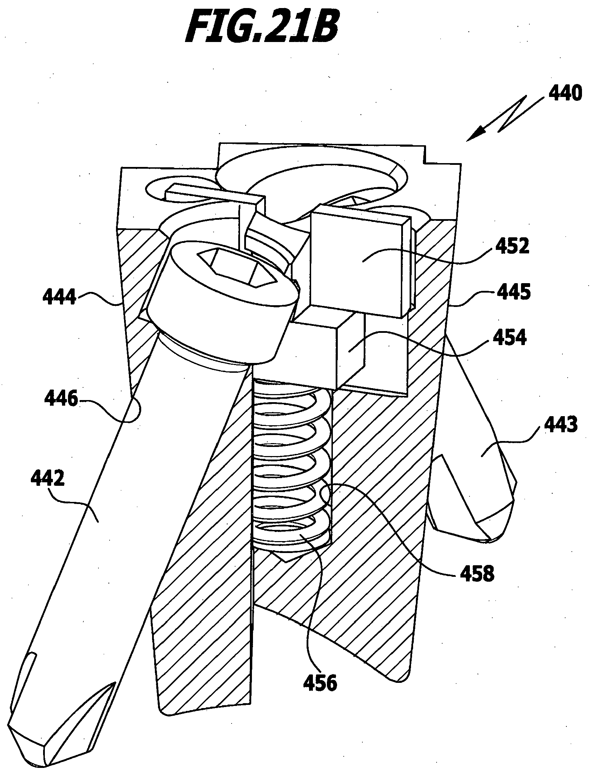

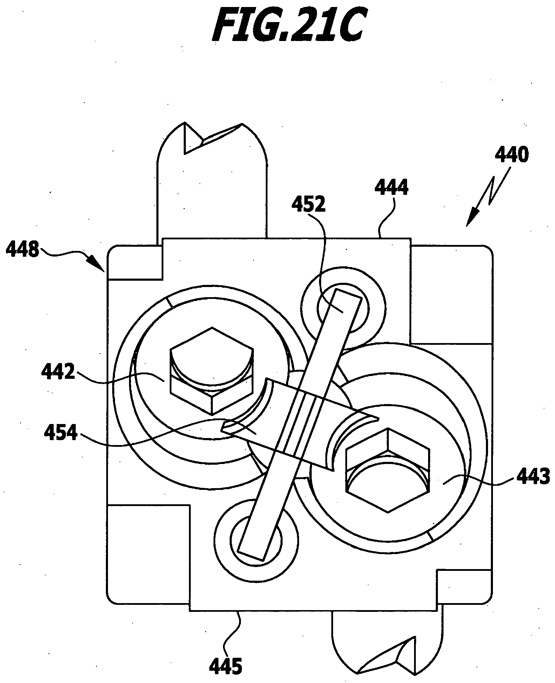

Figur 21 zeigt in unterschiedlichen Darstellungen ein Implantat 440, das ähnlich wie das Implantat 370 der Figur 19 über Schraubbolzen 442, 443 in der umgebenden Knochensubstanz fixiert wird. Zur Aufnahme und Führung der Schraubbolzen 442, 443 weist das Implantat 440 spitz gegenüber Kontaktflächen 444, 445 verlaufende Durchgangsöffnungen 446, 447 auf, die ausgehend von einem dorsalen Endbereich 448 des Implantats 440 an den Kontaktflächen 444, 445 austreten und dort das Eindringen der Schraubbolzen 442, 443 in die umgebende Knochensubstanz ermöglichen.

Um zu verhindern, dass sich die Schraubbolzen im Laufe der Zeit lockern können, ist eine Verriegelungsvorrichtung 450 vorgesehen, welche einen Schieber 452, ein Verriegelungselement 454 sowie eine Schraubenfeder 456 umfasst. Zur Aufnahme der Verriegelungsvorrichtung 450 ist in dem Implantat 440 eine zentrale, vom dorsalen Ende ausgehende Sacklochbohrung 458 vorgesehen, in der im montierten Zustand die Feder 456 aufgenommen ist. Oberhalb der Sacklochbohrung 458 ist eine Aufnahme für das Verriegelungselement 454 sowie den Schieber 452 vorgesehen, die diese führend hält. Beim Einsetzen des Implantats 440 in einen Schnittspalt eines Wirbelbogens wird vorzugsweise ein Halte- oder Einführinstrument verwendet, das am dorsalen Endbereich 448 des Implantats 440 befestigt wird.

Vorzugsweise wird mit der Befestigung des Einführ- bzw. Halteinstruments der Schieber 452 nach unten, d.h. gegen den ventralen Endbereich des Implantats 440, gedrückt, wobei gleichzeitig das Verriegelungselement 454 aus seiner aktiven Verriegelungsstellung heraus geschoben wird.

Nach dem Einsetzen des Implantats im Schnittspalt des Wirbelbogens wird, während das Halteinstrument noch am Implantat 440 befestigt ist, der Schraubbolzen 442 und der Schraubbolzen 443 in das umgebende Knochenmaterial eingeschraubt. Nachdem so das Implantat 440 in dem Schnittspalt des Wirbelbogens fixiert ist, kann das Halte- und Einführinstrument vom Implantat 440 getrennt werden, worauf dann die Verriegelungsvorrichtung 450 aktiviert wird und dabei das Verriegelungselement 454 durch die Feder 456 zusammen mit dem Schieber 452 in Richtung zum dorsalen Endbereich 448 verschoben wird. Das Verriegelungselement 454 gleitet dabei in eine dorsalseitige Endposition, in der es das Lösen der Schraubenbolzen 442, 443 blockiert, wie dies im Einzelnen in den Abbildungen 21B und 21C in perspektivischer bzw. Draufsicht dargestellt ist.

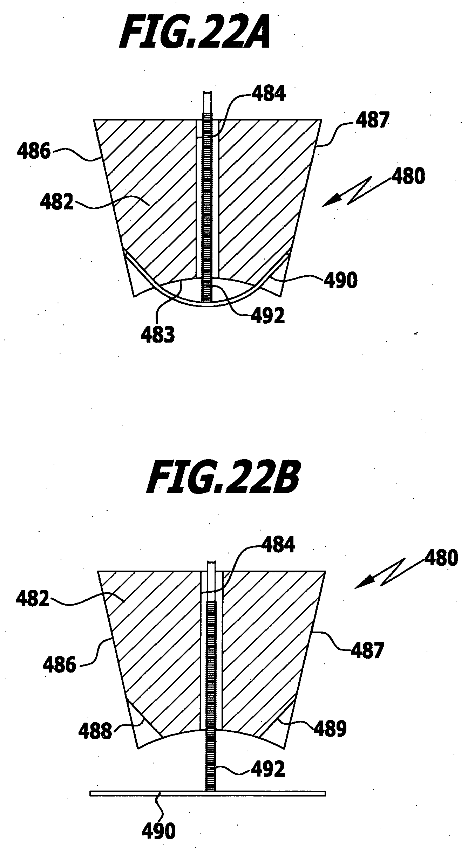

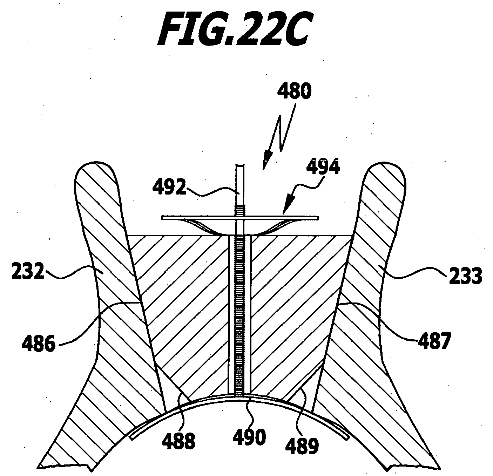

Eine weitere Variante eines Implantats ist in Figur 22 in den Abbildungen A bis C dargestellt, wobei hier eine weitere Variante zur Fixierung des Implantats 480 in seiner Position im Schnittspalt eines Wirbelbogens erläutert werden soll.

Der Implantatkörper 482 des Implantats 480 weist eine Längsbohrung 484 auf, die im Wesentlichen vom dorsalen zum ventralen Endbereich des Implantats 480 führt und im Wesentlichen mittig zwischen den keilförmig angeordneten Kontaktoberflächen 486, 487 angeordnet ist.

Der Implantatkörper 482 weist an seinem ventralen Ende neben einer in den vorgenannten Ausführungsformen bereits beschriebenen Einbuchtung 483 zur Vergrößerung des Volumens seitens des Spinalkanals benachbart zu seinen Kontaktflächen 486, 487 Rücksprünge 488, 489 auf.

Als Halteelement verwendet das Implantat 480 ein flächiges Federelement 490, welches beispielsweise streifenförmig ausgebildet sein kann. In der Mitte des Halteelements 490 ist ein Stift 492 fixiert, der den Implantatkörper 482 bzw. dessen Längsbohrung 484 dorsal durchsetzt.

Im einbaufertigen Zustand weist das Implantat 480 das Halteelement 490 in der in Abbildung A der Figur 22 gezeigten Konfiguration auf, bei der die freien Enden des Halteelements 490 in die Rücksprünge 488, 489 zurückgebogen sind und dort federnd eingreifen.

Im eingesetzten Zustand des Implantats 480 wird dann über den Stift 492 die Fixierung des Halteelements 490 in den Rücksprüngen 488, 489 gelöst, so dass sich dann das Halteelement 490 entfalten und eine im Wesentlichen ebene Konfiguration annehmen kann, wie dies in der Darstellung B der Figur 22 veranschaulicht ist.

Zum Fixieren des Implantats 480 im Schnittspalt eines Wirbelbogens wird dann das Halteelement 492 in dorsaler Richtung gezogen und in dieser Stellung dann mit einem Halteelement 494, wie es beispielsweise in der Figur 17 B im Einzelnen dargestellt ist, an der Oberseite des Implantats 480 fixiert. Nunmehr legt sich das Federelement 490 am Inneren der Lamina des Wirbels beidseits des Schnittspalts und an der Unterseite, der ventralen Seite des Implantats 480, an und sorgt so für eine Fixierung des Implantats im Schnittspalt des behandelten Wirbels, wie dies in Abbildung C der Figur 22 gezeigt ist.

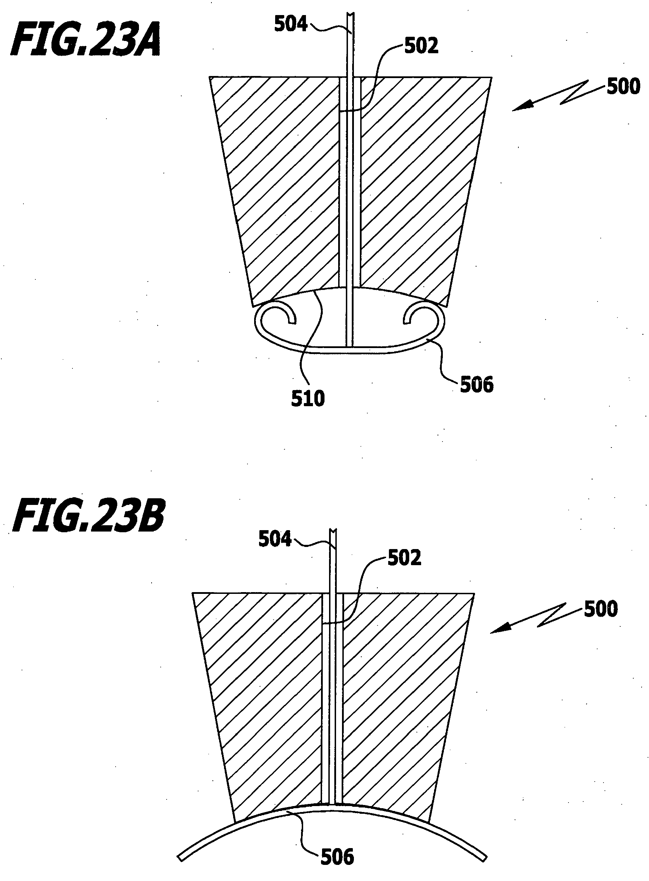

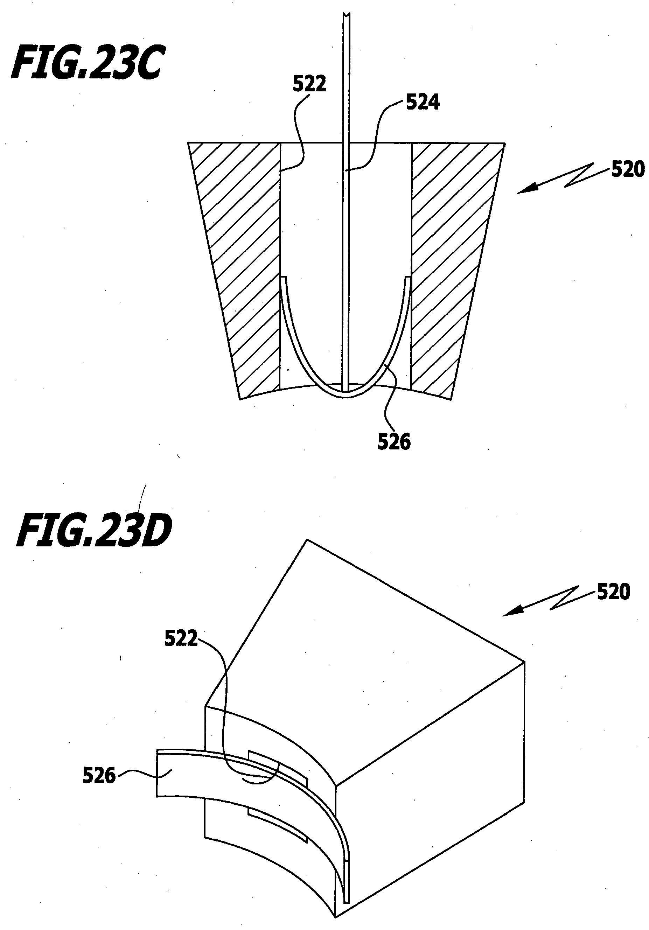

Die Figur 23 zeigt zwei Varianten des Implantats 480. In den Figuren 23A und 23B ist ein Implantat 500 gezeigt, das eine zentrale Bohrung 502 aufweiset, die vom dorsalen Endbereich bis zum ventralen Endbereich des Implantats 500 reicht.

Diese Durchgangsöffnung 502 nimmt einen Stift 504 verschieblich auf, der an seinem ventralen Ende ein Halteelement 506 in Form eines Federblechstreifens mittig hält. Im für die Implantation vorbereiteten Zustand des Implantats 500 ist das Federblech 506 mit seinen beiden freien Enden eingerollt gegen eine ventrale eingebuchtete Fläche 510 des Implantats 500 gehalten. Nachdem das Implantat 500 in den Schnittspalt eines Wirbelbogens eingesetzt ist, wird der Stift 504, ähnlich wie dies im Zusammenhang mit der Figur 22 beschrieben wurde, nach unten gedrückt, so dass sich das Federblech 506 entfalten kann. Danach wird der Stift 504 in dorsale Richtung gezogen, so dass das Federblech 506 in entfaltetem Zustand, wie in Figur 23B gezeigt, flächig an der eingebuchteten ventralen Seite 510 des Implantats 500 anliegt und darüber hinaussteht, so dass eine flächige Anlage an dem benachbarten Wirbelbogen (nicht gezeigt) resultiert, wie dies im Einzelnen bereits im Zusammenhang mit dem Implantat 480 in der Figur 22C gezeigt wurde.

Alternativ kann ein Implantat 520, wie in Figur 23C dargestellt, eine relativ große Durchgangsbohrung 522 aufweisen, in der nicht nur ein Haltestift 524 aufgenommen ist, sondern auch ein U-förmig zusammengebogenes Federblech 526, das mittig mit einem Ende des Stifts 524 verbunden ist.

Ist das Implantat 520 in den Schnittspalt eines Wirbelbogens eingesetzt, wird wiederum der Stift 524 nach unten, d.h. in ventrale Richtung, gedrückt, so dass sich dann das Federblech 526 entfalten kann. Danach wird der Stift 524 wieder in dorsale Richtung gezogen, so dass das Federblech 526 an der ventralen Seite des Implantats 520 zur Anlage kommt. Die am Implantat 520 beidseits überstehenden Bereiche des Federblechs 526 liegen dann an Teilen der Knochensubstanz des Wirbelbogens an.

Der Stift 524 kann in zurückgezogener Position, in der das Federblech 526 an der ventralen Seite des Implantats 520 anliegt, über ein Fixierelement ähnlich dem Fixierelement 494 der Figur 22C gehalten werden.

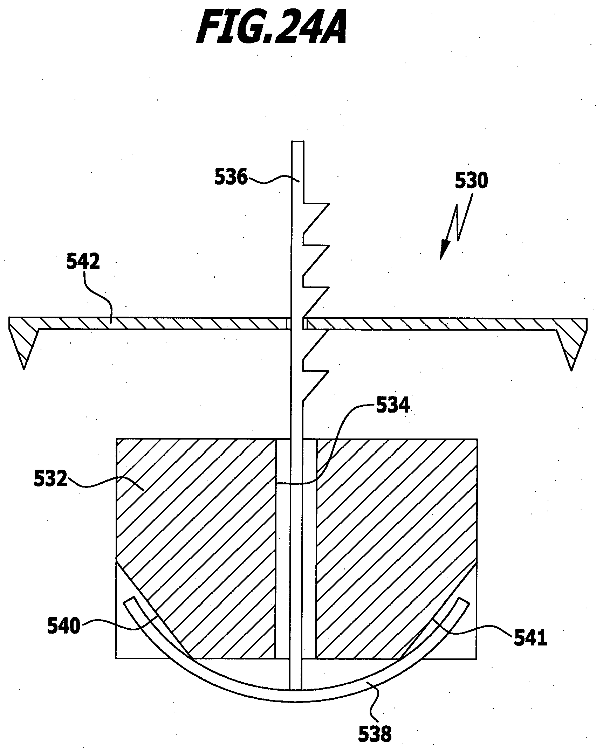

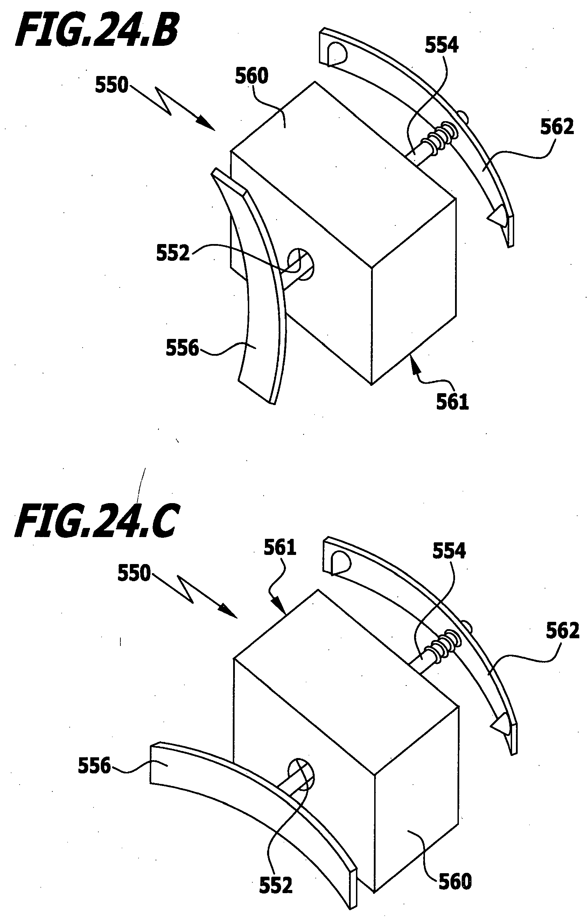

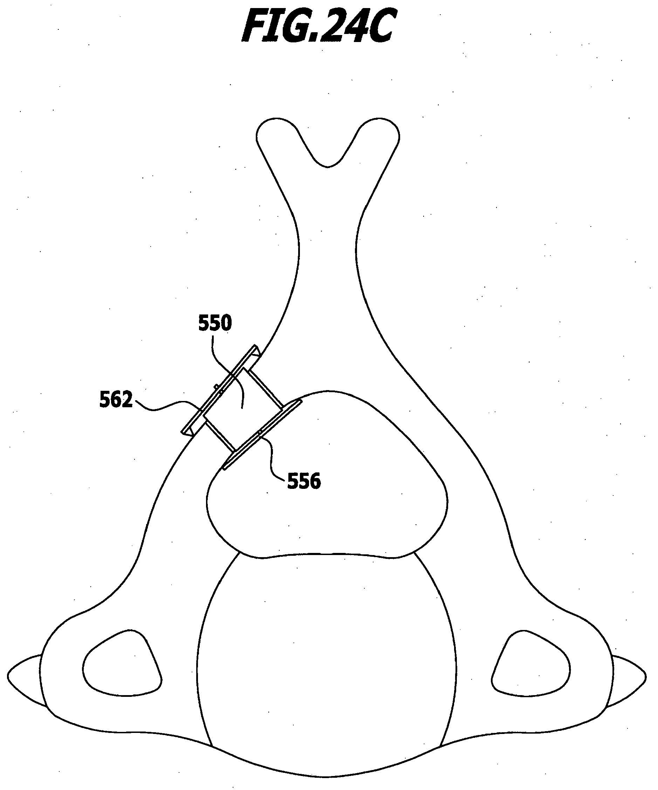

Zwei weitere alternative Ausführungsformen eines Implantats sind in der Figur 24 gezeigt. Bei dem Implantat 530 der Figur 24A weist der Implantatkörper 532 eine von der dorsalen zur ventralen Seite durchgehende Bohrung 534 auf. Ähnlich wie bei der Ausführungsform der Figur 22 hält ein Stift 536, der die Bohrung 534 von ventral nach dorsal durchsetzt, an seinem ventral gelegenen Ende ein Federblech 538. Das Federblech 538 ist zusammengebogen und in Rücksprüngen 540, 541 auf der ventralen Seite des Implantats 530 im zusammengebogenen Zustand fixiert. Wie bei der Ausführungsform der Figur 22 entfaltet sich das Federblech 538, wenn der Stift 536 in ventrale Richtung gedrückt wird, und kann dann durch Zurückziehen des Stiftes 536 zur flächigen Anlage an der ventralen Seite des Implantats 530 sowie den benachbarten Knochensubstanzanteilen des Wirbelbogens gebracht werden.

Anders als bei der Figur 22 wird jetzt der Haltestift 536 in seiner zurückgezogenen Position durch ein zweites, den Implantatkörper 530 übergreifendes Federblech 542 fixiert, welches den Implantatkörper beidseitig übergreift und mit Knochensubstanz auf der distalen Seite des Wirbelbogens liegt (vergleichbar der in Figur 24D dargestellten Situation).

Eine weitere Variante ist in den Figuren 24B und C gezeigt, bei der ein Implantat 550 wiederum eine Durchgangsöffnung 552 von der ventralen zur dorsalen Seite verlaufend aufweist, in der ein Haltestift 554 geführt ist. Der Haltestift 554 hält an seinem ventralen Ende einen Federblechstreifen 556, der, solange das Implantat 550 noch nicht in den Schnittspalt eines Wirbelbogens eingesetzt ist, in sagittaler Richtung ausgerichtet ist. Sobald das Implantat im Schnittspalt eines Wirbelbogens eingesetzt ist, wird das Federblech 556 um ca. 90° gedreht, so dass es dann die Kontaktflächen 560, 561 des Implantats 550 überragt und nachfolgend, nachdem der Stift 554 in dorsaler Richtung zurückgezogen ist, an der Knochensubstanz des Wirbelbogens in Anlage gebracht werden kann.

Auf der dorsalen Seite trägt der Haltestift 554 ein Sicherungselement 562, das ebenfalls die Kontaktflächen 560, 561 des Implantats 550 übergreift, so dass eine Einbausituation des Implantats im Wirbelbogen resultiert, wie sie in Figur 24D dargestellt ist.

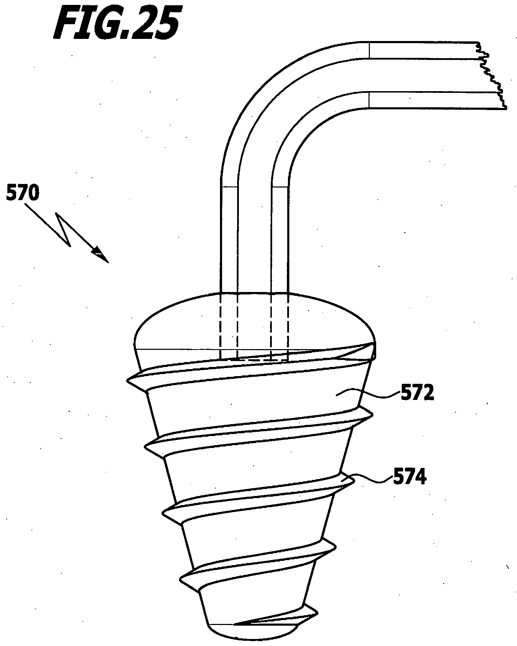

Figur 25 zeigt eine weitere Ausführungsform eines Implantats 570 mit einem konischen Implantatkörper 572, der an seiner Außenoberfläche ein Schraubgewinde 574 aufweist. Anders als bei den bisher beschriebenen Implantaten benötigt das Implantat 570 beim Einsetzen keinen aufgeweiteten Schnittspalt. Vorteilhaft ist, wenn der beim Durchtrennen der Lamina geschaffene Schnittspalt dorsal in den Schnittflächen gegenüber liegende Rücksprünge umfasst, in die zunächst der ventrale Endbereich des Implantats 570 zentrierend aufgenommen wird.

Beim nachfolgenden Einschrauben des Implantats 570 in den Schnittspalt schneidet das Gewinde 574 ein Gegengewinde in die Knochensubstanz ein. Aufgrund der konischen Form des Implantatkörpers 572 wird der Schnittspalt beim Einschrauben sukzessive aufgeweitet, so dass sich die Laminaabschnitte allmählich elastisch/plastisch verformen.

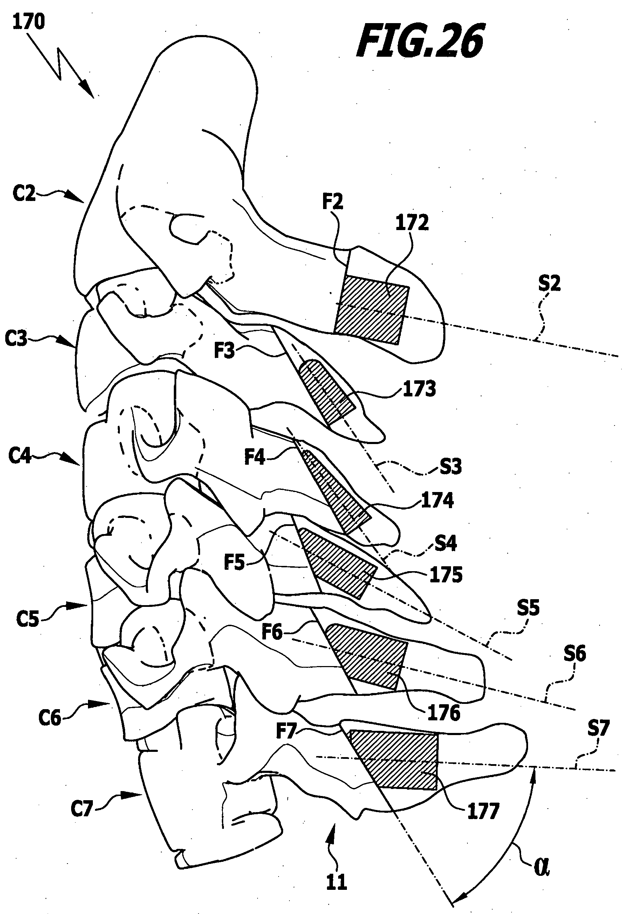

In der Figur 26 ist eine Seitenansicht in teilweise (entlang der Schnittspalte 20) aufgebrochener Form eines Halswirbelsäulenabschnitts 580 gezeigt, bei dem die Halswirbel C2 bis C7 jeweils mit einem Implantatkörper 582, 583, 584, 585, 586, 587 in aufgeweitetem Zustand des Wirbelkanals gehalten sind. Je nach Form und Größe des Dornfortsatzes der jeweiligen Halswirbel C2 bis C7 sind unterschiedlich große und unterschiedlich gestaltete Implantatkörper 582 bis 587 verwendet, die mit ihrer Längsachse jeweils im Wesentlichen parallel zur Längsachse S2, S3, S4, S5, S6, S7 des Dornfortsatzes ausgerichtet eingesetzt sind.

Die Hinterwand des Rückenmarkskanals (Spinalkanal 11) ist in den Halswirbeln C2 bis C7 mit F2 bis F7 bezeichnet. Sie stellt jeweils die dorsale Begrenzung des Rückenmarkskanals und die anteriore Begrenzung der Lamina dar.

Bei einer optimalen Anpassung der Implantatkörper an den jeweiligen Wirbel wird z.B. für den C2-Wirbel ein großer, rautenförmiger bis rechteckiger Querschnitt notwendig, die C3- und C4-Wirbel benötigen einen langen und eher flachen, keilförmigen Querschnitt, während für die C6- und C7-Wirbel eher kurze und dickere Keilformen der Implantatkörper 586, 587 notwendig sind.

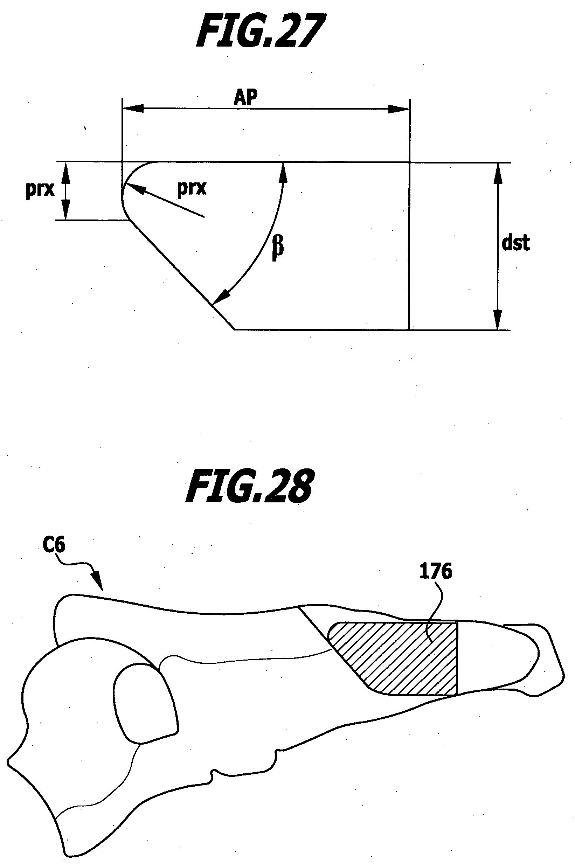

Die einzelnen Parameter, die diese Implantatkörper charakterisieren, sind in der Figur 27 gezeigt und zahlenmäßig beispielhaft in der Tabelle 2 angegeben.

**Tabelle 2**

| | **Breite dst** | **Länge AP** | **Schräge β [°]** | **Restbreite prx** | **Radius prx** | **α [°]** |
|---|---|---|---|---|---|---|
| C1 | - | - | - | - | - | - |
| C2 | 8,381 | 10,324 | - | - | - | 72,61 |
| C3 | 4,611 | 9,686 | 16,51 | 1,508 | 1,508 | 33,4 |
| C4 | 4,031 | 12,963 | 12,96 | 1,682 | 0,841 | 36,41 |
| C5 | 5,249 | 12,963 | 39,83 | 1,015 | 0,58 | 55,24 |
| C6 | 7,018 | 11,629 | 47,65 | 2,407 | 1,479 | 69,84 |
| C7 | 7,917 | 12,325 | 56,48 | 2,088 | - | 81,19 |

Der Winkel α ist als zwischen der Hinterwand F2 bis F7 (allgemein Fi) mit der jeweils zugehörigen Längsachse S2 bis S7 (allgemein Si) gebildeter Winkel definiert.

Bei der Aufweitung des Schnittspalts 20 wird vorzugsweise ein Distraktionswerkzeug vom Chirurgen von cranialer Position aus in den Schnittspalt eingeführt. Vorzugsweise werden hierzu Distraktionswerkzeuge verwendet, welche an ihrem distalen Ende nach außen abstehende Flansche oder Rippen aufweisen, die dabei an der Hinterwand Fi geführt werden. Die Hinterwand Fi dient dabei als Tiefenanschlag.

Beim Einsetzen des Implantats mit einem Einsetzwerkzeug kann das im Schnittspalt positionierte Distraktionswerkzeug und/oder die Hinterwand Fi des Spinalkanals zur Ausrichtung entlang der Längsachse Si des jeweiligen Dornfortsatzes dienen, z.B. wenn das im Schnittspalt eingesetzte Implantat um den vom Distraktionswerkzeug gebildeten Anschlag gedreht wird.

Die Figur 28 zeigt am Beispiel des C6-Implantats 586 dessen Einbausituation im Schnittspalt des Dornfortsatzes des C6-Wirbels in aufgebrochener Darstellung des Spinalkanals.

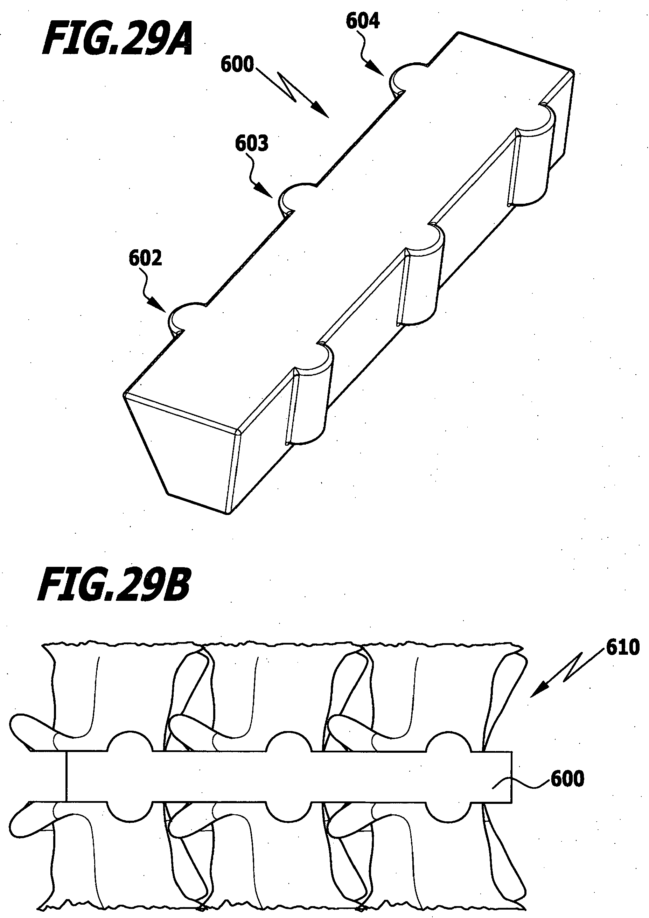

Figur 29 zeigt eine erste Ausführungsform eines Mehrfachimplantats 600, das geeignet ist, in in aufeinanderfolgende Wirbel eingebrachte Schnittspalte als ein Implantat eingesetzt zu werden. Die Ausformung des Mehrfachimplantats 600 entspricht im Wesentlichen dem Implantat 80 der Figur 6, bezogen auf den pro Wirbel vorgesehenen Implantatbereich 602, 603, 604 bzw. Schnittspalt. In der Draufsicht ist schematisch der implantierte Zustand in einem Abschnitt der Halswirbelsäule 610 beispielhaft gezeigt. Da die Implantatbereiche im Wesentlichen fest miteinander verbunden sind, führen sie so zu einer Stabilisierung und einer gewissen Immobilisierung des behandelten Wirbelsäulenabschnitts.

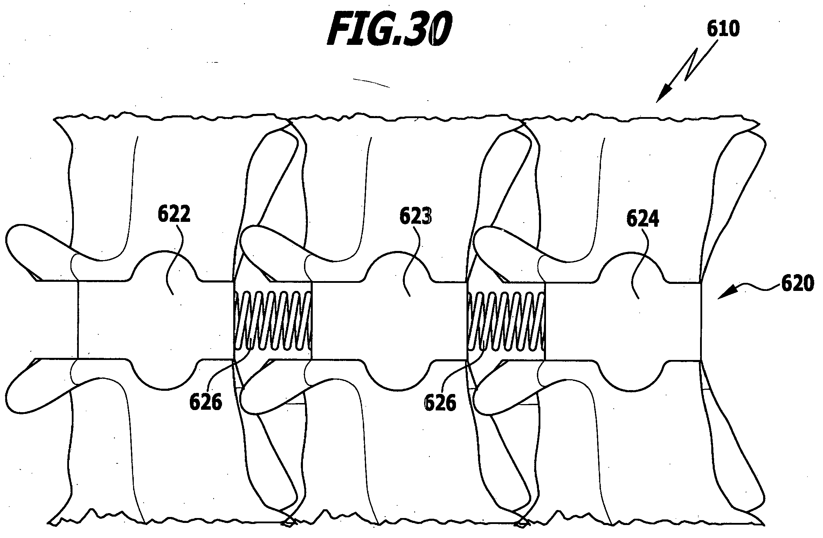

Figur 30 zeigt eine zweite Ausführungsform eines Mehrfachimplantats 620, bei dem die einzelnen Implantatabschnitte 622, 623, 624 nicht fest, sondern beweglich miteinander verbunden sind. Eine solche Verbindung kann insbesondere gelenkig und weiter bevorzugt, wie in Figur 30 gezeigt, in Sagittalrichtung elastisch, beispielsweise mittels Schraubenfedern 626, ausgestaltet sein. Auch hier findet eine Stabilisierung des behandelten Wirbelsäulenabschnitts 610 statt, allerdings ohne die in manchen Fällen unerwünschte Immobilisierung.

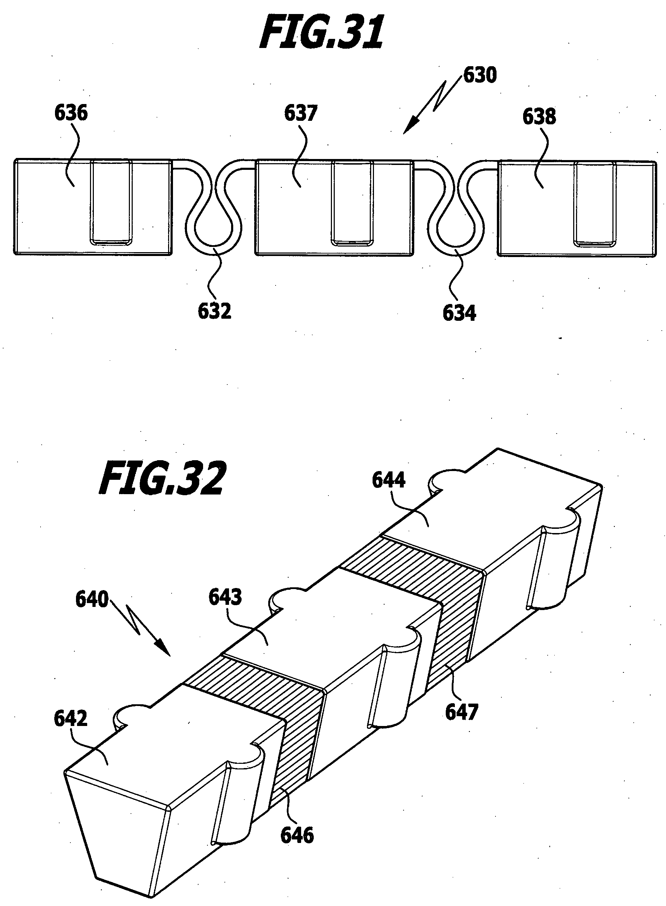

Eine Variante des Mehrfachimplantats 620 von Figur 30 ist in Figur 31 gezeigt. Das dort gezeigte Mehrfachimplantat 630 umfasst anstelle der Schraubenfedern 626 Blattfedern 632, 634, um die Implantatabschnitte 636, 637, 638 beweglich und federnd miteinander zu koppeln.

Eine weitere Variante des Mehrfachimplantats 620 der Figur 30 ist in Figur 32 gezeigt. Bei diesem Mehrfachimplantat 640 ist die elastische Verbindung zwischen den einzelnen Implantatsabschnitten 642, 643, 644 über Elastomerbrücken 646, 647 gelöst. Die Elastomerbrücken 646, 647 lassen sich in ihrer Elastizität so einstellen, dass die erwünschte Bewegungsfreiheit zwischen den einzelnen Wirbeln erhalten bleibt, jedoch unerwünscht große Bewegungen, die den Behandlungserfolg beeinträchtigen könnten, behindert oder gar verhindert werden.

## Patentansprüche

1. Implantat zur Verwendung in der Laminoplastie oder Laminektomie, bei der der Wirbelbogen eines Wirbels unter Bildung eines Schnittspalts durchtrennt oder partiell entfernt und das Implantat in den Schnittspalt eingesetzt wird, wobei der Schnittspalt von einander gegenüberliegenden Schnittflächen begrenzt ist, wobei das Implantat einen Implantatkörper mit zwei Kontaktflächen umfasst, welche keilförmig gegeneinander geneigt ausgebildet sind, wobei der Abstand der Kontaktflächen an einem dorsalen Endbereich des Implantatkörpers größer ist als am ventralen Endbereich, und wobei die Kontaktflächen im in den Schnittspalt eingesetzten Zustand an den Schnittflächen des Wirbelbogens anliegen, **dadurch gekennzeichnet, dass** der Implantatkörper Bohrungen aufweist, welche vom dorsalen Endbereich des Implantatkörpers ausgehend im spitzen Winkel zu den Kontaktflächen des keilförmigen Implantatkörpers ausgerichtet sind.

2. Implantat nach Anspruch 1, **dadurch gekennzeichnet, dass** an den Kontaktflächen des Implantatkörpers Führungselemente angeordnet sind,
wobei die Führungselemente optional im Wesentlichen als Vor- oder Rücksprünge ausgebildet sind, die insbesondere im Wesentlichen parallel zur Längsachse des keilförmigen Implantatkörpers ausgerichtet sind.

3. Implantat nach Anspruch 1 oder 2, **dadurch gekennzeichnet, dass** der Implantatkörper ein Führungselement an seinem ventralen Ende aufweist
und/oder dass der Implantatkörper ein Anschlagelement aufweist, welches an der Knochensubstanz zur Anlage kommt und die Einsetztiefe des Implantats im Schnittspalt auf einen vorgegebenen Wert begrenzt.

4. Implantat nach einem der Ansprüche 1 bis 3, **dadurch gekennzeichnet, dass** der Implantatkörper Längsbohrungen aufweist, die im Wesentlichen parallel zur Längsachse des keilförmigen Implantatkörpers ausgerichtet sind und zumindest auf der dorsalen Seite des Implantatkörpers frei zugänglich sind;
und/oder dass der Implantatkörper eine oder mehrere Querbohrungen aufweist, welche quer zur Längsrichtung des keilförmigen Implantatkörpers ausgerichtet sind und welche vorzugsweise Durchgangsbohrungen sind.

5. Implantat nach einem der Ansprüche 1 bis 4, **dadurch gekennzeichnet, dass** die Bohrungen des Implantatkörpers, welche vom dorsalen Endbereich des Implantatkörpers ausgehend im spitzen Winkel zu den Kontaktflächen des keilförmigen Implantatkörpers ausgerichtet sind, die Kontaktflächen durchsetzen.

6. Implantat nach einem der Ansprüche 1 bis 5, **dadurch gekennzeichnet, dass** das Implantat eine oder mehrere Schrauben, Bolzen und oder Dübel umfasst.

7. Implantat nach einem der Ansprüche 1 bis 6, **dadurch gekennzeichnet, dass** der Implantatkörper eine zentrale, zum dorsalen Endbereich hin offene Sacklochöffnung umfasst, in der eine Fixiervorrichtung gehalten ist, die in einer Endstellung einen Formschluss mit dem das Implantat im eingesetzten Zustand umgebenden Knochenmaterial herstellt,
wobei vorzugsweise die Fixiervorrichtung einen Schraubbolzen umfasst, dessen Durchmesser größer ist als der Abstand der Kontaktflächen am dorsalen Endbereich des Implantatkörpers.

8. Implantat nach Anspruch 7, **dadurch gekennzeichnet, dass** die Fixiervorrichtung ein oder mehrere dornartige Arretierelemente umfasst, welche von einer oder mehreren Betätigungsvorrichtungen aus einer Ruhelage in eine Arbeitslage überführbar sind, wobei die Spitzen der Arretierelemente in der Ruhelage innerhalb der Kontur des Impläntatkörpers angeordnet sind und in der Arbeitslage aus Öffnungen in den Kontaktflächen aus diesen in die umgebende Knochensubstanz heraustreten.

9. Implantat nach Anspruch 6, **dadurch gekennzeichnet, dass** der oder die Dübel plastifizierbar sind, vorzugsweise mittels Wärme, Ultraschall, UV-Licht oder HF-Strahlung,
oder alternativ dass der oder die Dübel heißklebefähig sind.

10. Implantat nach einem der Ansprüche 1 bis 9, **dadurch gekennzeichnet, dass** der Implantatkörper eine im Wesentlichen zentrale Durchgangsöffnung aufweist, welche vom dorsalen Endbereich zum ventralen Endbereich reicht,
wobei optional das Implantat eine Verankerungsvorrichtung umfasst, welche ein am ventralen Endbereich angeordnetes Flächenelement und eine am Flächenelement gehaltene Haltevorrichtung umfasst, wobei das Flächenelement in mindestens einer Richtung seiner Flächenausdehnung eine größere Breite aufweist als der Abstand der Kontaktflächen des Implantatkörpers am ventralen Endbereich, und wobei die Haltevorrichtung durch die zentrale Durchgangsöffnung zum dorsalen Endbereich verläuft und an diesem festlegbar ist,
und wobei vorzugsweise das Flächenelement aus einem federelastischen Material hergestellt ist.

11. Implantat nach Anspruch 10, **dadurch gekennzeichnet, dass** das Flächenelement in einer ersten Stellung elastisch verformt am oder im Implantatkörper gehalten ist, wobei die Flächenausdehnung des Flächenelements im elastisch verformten Zustand in Richtung des Abstandes der Kontaktflächen geringer ist als der Abstand der Kontaktflächen am ventralen Endbereich des Implantatkörpers.

12. Implantat nach einem der voranstehenden Ansprüche, **dadurch gekennzeichnet, dass** das Implantat am dorsalen Endbereich ein Griffelement aufweist.

13. Implantat nach einem der Ansprüche 1 bis 12, **dadurch gekennzeichnet, dass** der Implantatkörper an seinem ventralen Endbereich einen Abstand der keilförmig angeordneten Kontaktflächen aufweist, welcher ca. 5 mm bis ca. 15 mm beträgt.

14. Implantat nach einem der Ansprüche 1 bis 13, **dadurch gekennzeichnet, dass** die Kontaktflächen so ausgebildet sind, dass sie sich im Wesentlichen vollflächig an den Schnittflächen des Schnittspalts abstützen, und/oder dass die Kontaktflächen mit Ausnahme der die gegebenenfalls vorhandenen Vor- oder Rücksprünge enthaltenden Bereiche im Wesentlichen eben sind.

15. Implantat nach einem der Ansprüche 2 bis 14, **dadurch gekennzeichnet, dass** die Rücksprünge der Kontaktflächen als Nuten ausgebildet sind, welche insbesondere im Wesentlichen halbzylindrisch ausgebildet sind,
wobei vorzugsweise die Nuten im Wesentlichen parallel zur Längsrichtung des Dornfortsatzes verlaufend ausgebildet sind.

## Claims

1. Implant for use in a laminoplasty or laminectomy method in which the vertebral arch of a vertebra is cut through or partially removed, forming an incision gap, and the implant is inserted in the incision gap, wherein the incision gap is bounded by incision faces opposing each other,
wherein the implant comprises an implant body having two contact faces which are configured inclined relative to each other in the shape of a wedge, wherein the distance between the contact faces is larger at a dorsal end region of the implant body than at a ventral end region thereof, and wherein the contact faces in the inserted state in the incision gap contact the incision faces of the vertebral arch,
**characterized in that** the implant body has bores which, starting from the dorsal end region of the implant body, are oriented at an acute angle with respect to the contact faces of the wedge-shaped implant body.

2. Implant in accordance with claim 1, **characterized in that** the contact faces of the implant body have guide elements arranged thereat, wherein the guide elements are optionally essentially configured as projections or recesses which are particularly oriented substantially parallel to the longitudinal axis of the wedge-shaped implant body.

3. Implant in accordance with claim 1 or 2, **characterized in that** the implant body has a guide element at the ventral end thereof and/or that the implant body has a stop element which comes into contact with the bone substance and limits the insertion depth of the implant in the incision gap to a predetermined value.

4. Implant in accordance with any one of claims 1 to 3, **characterized in that** the implant body has longitudinal bores which are oriented substantially parallel to the longitudinal axis of the wedge-shaped implant body and are freely accessible at least on the dorsal side of the implant body;
and/or that the implant body has one or more transverse bores which are oriented transversely to the longitudinal axis of the wedge-shaped implant body and are preferably through-bores.

5. Implant in accordance with any one of claims 1 to 4, **characterized in that** the bores of the implant body which, starting from the dorsal end region of the implant body, are oriented at an acute angle with respect to the contact faces of the wedge-shaped implant body, extend through the contact faces.

6. Implant in accordance with any one of claims 1 to 5, **characterized in that** the implant comprises one or more screws, bolts and/or dowels.

7. Implant in accordance with any one of claims 1 to 6, **characterized in that** the implant body comprises a central blind hole opening which opens towards the dorsal end region, in which is held a fixation device which, when in an end position, establishes a form-locking engagement with the bone material surrounding the implant in the inserted state, wherein the fixation device comprises a screw bolt having a diameter that is larger than the distance between the contact faces at the dorsal end region of the implant body.

8. Implant in accordance with claim 7, **characterized in that** the fixation device comprises one or more spike-like fixation elements which are transitionable from an inoperative position to an operative position by one or more actuating devices, wherein the tips of the fixation elements in the inoperative position are arranged within the contour of the implant body and in the operative position emerge from openings in the contact faces into the surrounding bone substance.

9. Implant in accordance with claim 6, **characterized in that** the one or more dowels are plastifiable, preferably by heat, ultrasound, UV light or HF radiation,
or alternatively that the one or more dowels are heat-bondable.

10. Implant in accordance with any one of claims 1 to 9, **characterized in that** the implant body has a substantially central through-opening which extends from the dorsal end region to the ventral end region,
wherein optionally the implant comprises an anchoring device which comprises a sheet element arranged on the ventral end region and a holding device held on the sheet element, wherein the sheet element has in at least one direction of its extension a width that is larger than the distance between the contact faces of the implant body at the ventral end region, and wherein the holding device extends through the central through-opening to the dorsal end region and is fixable thereat, and wherein preferably the sheet element is made of a resilient material.

11. Implant in accordance with claim 10, **characterized in that** the sheet element is, in a first position, held in an elastically deformed state at or in the implant body, wherein the extension of the sheet element in the elastically deformed state is in a direction of the distance between the contact faces smaller than the distance between the contact faces at the ventral end region of the implant body.

12. Implant in accordance with any one of the preceding claims, **characterized in that** the implant has a grip element at the dorsal end region thereof.

13. Implant in accordance with any one of claims 1 to 12, **characterized in that** the implant body has at its ventral end region a distance between the contact faces arranged in the shape of a wedge that is about 5 mm to about 15 mm.

14. Implant in accordance with any one of claims 1 to 13, **characterized in that** the contact faces are configured such that they are supported over essentially the entire area thereof on the incision faces of the incision gap,
and/or that the contact faces are substantially planar except for the areas containing the projections or recesses that may optionally be present.

15. Implant in accordance with any one of claims 2 to 14, **characterized in that** the recesses of the contact faces are configured as grooves which are in particular of substantially semi-cylindrical configuration, wherein the grooves are preferably configured to extend substantially parallel to the longitudinal direction of the spinous process.

## Revendications

1. Implant destiné à être utilisé dans la laminoplastie ou la laminectomie, pour laquelle l'arc vertébral d'une vertèbre est sectionné ou partiellement enlevé en formant un interstice de coupe, et l'implant est inséré ou implanté dans l'interstice de coupe, implant dans lequel l'interstice de coupe est délimité par deux surfaces de coupe mutuellement opposées,
dans lequel l'implant comprend un corps d'implant avec deux surfaces de contact, qui sont d'une configuration inclinée en forme de coin l'une par rapport à l'autre, dans lequel la distance d'espacement des surfaces de contact est plus grande au niveau d'une zone d'extrémité dorsale du corps d'implant qu'au niveau de la zone d'extrémité ventrale,
et dans lequel les surfaces de contact, dans l'état implanté dans l'interstice de coupe, s'appuient contre les surfaces de coupe de l'arc vertébral,
**caractérisé en ce que** le corps d'implant comporte des alésages, qui sont orientés, en étant issus de la zone d'extrémité dorsale du corps d'implant, de manière à former un angle aigu par rapport aux surfaces de contact du corps d'implant en forme de coin.

2. Implant selon la revendication 1, **caractérisé en ce que** sur les surfaces de contact du corps d'implant sont agencés des éléments de guidage, les éléments de guidage étant réalisés, de manière optionnelle, sensiblement en tant que protubérances ou retraits, qui sont en particulier orientés sensiblement de manière parallèle à l'axe longitudinal du corps d'implant en forme de coin.

3. Implant selon la revendication 1 ou la revendication 2, **caractérisé en ce que** le corps d'implant présente un élément de guidage à son extrémité ventrale,
et/ou **en ce que** le corps d'implant présente un élément de butée, qui vient s'appuyer contre la substance osseuse et limite la profondeur d'insertion de l'implant dans l'interstice de coupe, à une valeur prescrite.

4. Implant selon l'une des revendications 1 à 3, **caractérisé en ce que** le corps d'implant présente des alésages longitudinaux, qui sont orientés sensiblement de manière parallèle à l'axe longitudinal du corps d'implant en forme de coin, et sont librement accessibles au moins sur le côté dorsal du corps d'implant,
et/ou **en ce que** le corps d'implant présente un ou plusieurs alésages transversaux, qui sont orientés transversalement à la direction longitudinale du corps d'implant en forme de coin, et qui de préférence sont des alésages de passage.

5. Implant selon l'une des revendications 1 à 4, **caractérisé en ce que** les alésages du corps d'implant, qui sont orientés, en étant issus de la zone d'extrémité dorsale du corps d'implant, de manière à former un angle aigu par rapport aux surfaces de contact du corps d'implant en forme de coin, traversent les surfaces de contact.

6. Implant selon l'une des revendications 1 à 5, **caractérisé en ce que** l'implant comprend une ou plusieurs vis, broches et/ou chevilles.

7. Implant selon l'une des revendications 1 à 6, **caractérisé en ce que** le corps d'implant comporte une ouverture borgne centrale, ouverte vers la zone d'extrémité dorsale, et dans laquelle est maintenu un dispositif de fixation, qui, dans une position finale, établit une liaison par complémentarité de formes avec le matériau osseux entourant l'implant dans l'état implanté, le dispositif de fixation comprenant de préférence une broche à visser dont le diamètre est plus grand que la distance d'espacement des surfaces de contact au niveau de la zone d'extrémité dorsale du corps d'implant.

8. Implant selon la revendication 7, **caractérisé en ce que** le dispositif de fixation comprend un ou plusieurs éléments d'arrêt en forme d'épine, qui peuvent être transférés d'une position de repos à une position de travail par un ou plusieurs dispositifs d'actionnement, les pointes des éléments d'arrêt étant agencées, dans la position de repos, à l'intérieur du contour du corps d'implant, et sortant, dans la position de travail, des surfaces de contact par des ouvertures dans celles-ci, pour pénétrer dans la substance osseuse environnante.

9. Implant selon la revendication 6, **caractérisé en ce que** la ou les chevilles peuvent être plastifiées, de préférence au moyen de chaleur, d'ultrasons, de lumière UV ou de rayonnement HF,
ou **en ce qu'**en variante les chevilles sont adhésives à chaud.

10. Implant selon l'une des revendications 1 à 9, **caractérisé en ce que** le corps d'implant présente une ouverture de passage sensiblement centrale, qui s'étend de la zone d'extrémité dorsale jusqu'à la zone d'extrémité ventrale,
l'implant comporte de manière optionnelle, un dispositif d'ancrage comprenant un élément mince de surface agencé au niveau de la zone ventrale et un dispositif de maintien supporté par l'élément mince de surface, l'élément mince de surface présentant, dans au moins une direction de son étendue de surface, une largeur plus grande que la distance d'espacement des surfaces de contact du corps d'implant au niveau de la zone d'extrémité ventrale, et le dispositif de maintien s'étendant à travers l'ouverture de passage centrale vers la zone d'extrémité dorsale et pouvant être fixé à celle-ci,
et l'élément mince de surface est de préférence réalisé en un matériau à élasticité de ressort.

11. Implant selon la revendication 10, **caractérisé en ce que** l'élément mince de surface est, dans une première position, maintenu sur ou dans le corps d'implant en étant déformé élastiquement, l'étendue de surface de l'élément mince de surface étant, dans l'état de déformation élastique, plus faible dans la direction de la distance d'espacement des surfaces de contact, que la distance d'espacement de ces surfaces de contact au niveau de la zone d'extrémité ventrale du corps d'implant.

12. Implant selon l'une des revendications précédentes, **caractérisé en ce que** l'implant présente un élément de préhension, dans la zone d'extrémité dorsale.

13. Implant selon l'une des revendications 1 à 12, **caractérisé en ce que** le corps d'implant présente au niveau de sa zone d'extrémité ventrale, une distance d'espacement des surfaces de contact agencées en forme de coin, d'une valeur d'environ 5 mm à environ 15 mm.

14. Implant selon l'une des revendications 1 à 13, **caractérisé en ce que** les surfaces de contact sont configurées de manière à s'appuyer sensiblement selon toute leur surface sur les surfaces de coupe de l'interstice de coupe, et/ou **en ce que** les surfaces de contact sont sensiblement planes, exception faite des zones renfermant les protubérances et retraits existant éventuellement.

15. Implant selon l'une des revendications 2 à 14, **caractérisé en ce que** les retraits des surfaces de contact sont réalisés sous forme de rainures, qui sont notamment de configuration sensiblement demi-cylindrique, les rainures étant de préférence réalisées de manière à s'étendre sensiblement de façon parallèle à la direction longitudinale de l'apophyse épineuse.
